# EUROPEAN PATENT APPLICATION

(11) **EP 3 570 035 A1**
(43) Date of publication of application: **20.11.2019**
(21) Application number: 18172316.4
(22) Date of filing: 15.05.2018
(51) Int. Cl.: G01N 33/68

(54) **DETERMINING PLATELET REACTIVITY**

(71) Applicant: Medizinische Universität Wien, 1090 Wien (AT)
(72) Inventor: ZELLNER, Maria, 1190 Wien (AT); SCHMIDT, Georg, 1090 Wien (AT); JILMA, Bernd, 1090 Wien (AT)
(74) Representative: Loidl, Manuela Bettina

(57) **Abstract**

This invention refers to the field of determination of platelet reactivity using biomarkers to identify subjects at risk of or having a vascular disease or a bleeding disorder and to monitor treatment success of subjects receiving treatment for a vascular disease or a bleeding disorder.

## Description

### FIELD OF THE INVENTION

This invention lies in the field of determination of platelet reactivity using biomarkers to identify subjects at risk of or having a vascular disease or a bleeding disorder and to monitor treatment success of subjects receiving treatment for a vascular disease or a bleeding disorder.

### BACKGROUND OF THE INVENTION

Platelets represent a decisive factor in (cardio) vascular diseases, the leading cause of death in the industrialized world. Indeed, inappropriate platelet reactivity and subsequent thrombus formation contribute to as many as 610,000 deaths per year in the USA.¹ Platelets are preserved in a sensitive balance between upholding adequate blood-flow and promoting blood clotting in the case of bleeding. Upon vessel injury, platelets instantly become activated, leading to granule release, aggregation, and plug formation. Although much is known about factors resulting in platelet activation, the molecular characteristics maintaining platelets in their balanced state are not well characterized. Since the triggering of unscheduled platelet activation represents such a serious health hazard, it is necessary to explore biomarkers ensuring platelets' activation status for their monitoring in health and disease.

Major *in vivo* triggers for activation include binding of sub-endothelial collagen as well as other platelet agonists, like adenosine-diphosphate (ADP) and thrombin, to their cognate receptors.^{2,3} Under balanced physiological conditions, endothelial cells produce prostacyclin (PGI₂) and nitric oxide, thereby keeping platelets silenced. Both mediators induce the synthesis of cyclic nucleotides (cAMP, cGMP), which activate protein kinases A and G (PKA, PKG) in platelets. These serine/threonine kinases phosphorylate particular proteins to counteract the activation of platelets.⁴ Platelet activation and thrombus formation can, therefore, be regarded as the predominance of either pro-thrombotic factors or a reduction of endogenous inhibitors.⁵ An impairment⁶ of platelet-inactivation mechanisms, or resistance⁷ to them, may result in hyper-reactive platelets and spontaneous generation of platelet-dependent thrombosis.^{8,9} Accordingly, typical platelet activation markers like P-selectin (CD62P) and activated integrin α_{IIb}β₃ (CD41/CD61) are significantly changed under these conditions and are thus used to assess the platelet activation status.¹⁰⁻¹² However, whether increased platelet reactivity results from an endogenous activation or from an insufficient inactivation remains largely unknown.

To investigate how the platelets' protein repertoire is affected by activation and inactivation in a comprehensive and unbiased manner, platelet proteomics may be applied. Several *in vitro* platelet proteomic studies have shed light on the complexity of signal propagation induced upon activation with thrombin receptor-activating peptide-6 (TRAP-6)¹³, ADP¹⁴, collagen-related peptide¹⁵ or via the C-type lectin-like receptor 2.¹⁶ Although an insufficient inactivation of platelets is also a significant feature in platelet-related thrombotic disorders, the first proteomic studies regarding this have only recently been published.¹⁷ There, the dynamic short-term inactivation platelet phospho-proteome was analyzed via stimulation of the cyclic adenosine monophosphate/protein kinase A (cAMP/PKA) pathway¹⁷ or with additional activation by ADP.¹⁸ Nevertheless, a direct comparison of the platelet proteome response upon both activation and inactivation might also uncover pathophysiological processes in thrombotic diseases. Understanding abnormal platelet function from the perspective of both main regulatory systems is essential to establish sensitive and robust biomarkers and to be able to target platelets pharmaceutically according to the underlying pathology and thus to identify new therapeutic options.

There is an urgent need in the field for reliable biomarkers to identify abnormal platelet reactivity to diagnose platelet activation and/or inactivation disorders and to monitor platelet activation or inactivation potential and reactivity in patients with vascular diseases or bleeding disorders.

In view of the above, it is an object of the invention to provide novel, more robust biomarkers for the diagnosis and treatment success monitoring of vascular diseases and bleeding disorders.

### SUMMARY OF THE INVENTION

The object is solved by the invention as claimed.

According to the invention there is provided an *in vitro* method of identifying abnormal platelet reactivity potential of a subject, comprising the steps of:
i) providing a sample comprising the platelet proteome of said subject, and
ii) measuring the phosphorylation level of at least one member of the cAMP/ protein kinase A (PKA)-pathway and comparing the phosphorylation level of said at least one cAMP/PKA-pathway member with the phosphorylation level of the corresponding at least one cAMP/PKA-pathway member in a reference sample, and/or
iii) measuring the phosphorylation level of at least one member of the phospholipase C/protein kinase C (PKC)-pathway and comparing the phosphorylation level of said at least one phospholipase C/PKC-pathway member with the phosphorylation level of the corresponding at least one phospholipase C/PKC-pathway member in a reference sample,
wherein a difference in the phosphorylation level of at least 1.5 fold in ii) and/or iii) is indicative of abnormal platelet reactivity potential.

According to a specific aspect of the invention provided herein, the abnormal platelet reactivity detected by the method provided herein is correlated with vascular disease or bleeding disorders. Specifically, an increase in platelet reactivity potential is indicative of a vascular disease and a decrease in platelet reactivity potential is indicative of a bleeding disorder. Typically, subjects identified as having increased platelet reactivity potential are subjected to treatment for vascular disease, preferably using anticoagulants and/or antiplatelet agents. Typically, subjects identified as having decreased platelet reactivity potential are subjected to treatment for bleeding disorder, preferably using substitution treatment and/or clotting factor concentrate.

Specifically, the subject is a human or non-human mammal. Preferably non-human mammalian subjects are selected from the group consisting of dogs, cats, horses, cattle, pigs, goats, sheep, camels, guinea pigs, mice, rats and rabbits and non-human primates, e.g. monkeys such as cynomolgous or rhesus monkeys or chimpanzees. Even more preferred, the subject is a human subject.

Specifically, subjects can be patients suffering from a vascular disorder or bleeding disorder or are subjects suspected to be suffering or being at risk of suffering from a vascular disorder or bleeding disorder.

Specifically, the phosphorylation level shows a difference compared to the reference sample of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold. Preferably, the phosphorylation level shows a difference compared to the reference sample of at least 1.5, 2.0 or 3.0 fold.

According to a specific embodiment of the method provided herein, the at least one member of the cAMP/PKA-pathway is selected from the group consisting of LIM and SH3 domain protein 1 (LASP1), Src kinase-associated phosphoprotein 2 (SKAP2), Zyxin (ZYX), regulator of G-protein signaling 18 (RGS18), Caldesmon 1 (CALD1) and Nucleosome assembly protein 1-like 1 (NAP1L1).

Specifically, in the case of diagnosis a decrease, compared to a reference sample, of at least 1.5 fold in the phosphorylation level of any one or more of LASP1, SKAP2, ZYX and RGS18 indicates increased platelet reactivity potential. Specifically, when platelet reactivity potential is increased, the phosphorylation level of LASP1, SKAP2, ZYX and/or RGS18 shows a decrease, compared to the reference sample, of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold. To the contrary, specifically, when the phosphorylation level of LASP1, SKAP2, ZYX and/or RGS18 shows an increase, compared to the reference sample, of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, or 6 fold it indicates decreased platelet reactivity potential.

Specifically, in the case of diagnosis an increase, compared to a reference sample, of at least 1.5 fold in the phosphorylation level of CALD1 and/or NAP1L1 indicates increased platelet reactivity potential. Specifically, when platelet reactivity potential is increased the phosphorylation level of CALD1 and/or NAP1L1 shows an increase compared to the reference sample of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold. To the contrary, specifically, when the phosphorylation level of CALD1 and/or NAP1L1 shows a decrease, compared to the reference sample, of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold it indicates decreased platelet reactivity potential.

According to a further specific embodiment of the method provided herein, the at least one member of the phospholipase C/PKC-pathway is selected from the group consisting of pleckstrin (PLEK) and integrin-linked protein kinase (ILK).

Specifically, in the case of diagnosis an increase, compared to a reference sample, in the phosphorylation level of PLEK is indicative of increased platelet activation, whereas a decrease in the phosphorylation level of PLEK is indicative of decreased platelet activation. Specifically, in the case of diagnosis a decrease, compared to a reference sample, in the phosphorylation level of ILK is indicative of increased platelet activation, whereas an increase in the phosphorylation level of ILK is indicative of decreased platelet activation.

Specifically, an increase of at least 1.5 fold in the phosphorylation level of PLEK indicates increased platelet reactivity potential. Specifically, when platelet reactivity potential is increased, the phosphorylation level of PLEK shows an increase, compared to the reference sample, of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold. Contrary, when platelet reactivity potential is decreased, the phosphorylation level of PLEK shows a decrease, compared to the reference sample, of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold.

Specifically, a decrease of at least 1.5 fold in the phosphorylation level of ILK indicates increased platelet reactivity. Specifically, when platelet reactivity potential is increased, the phosphorylation level of ILK shows a decrease of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold. Contrary, when platelet reactivity potential is decreased, the phosphorylation level of ILK shows an increase of at least 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0 or 6 fold.

According to a specific embodiment of the method provided herein, the phosphorylation levels of at least one member of the cAMP/PKA-pathway and at least one member of the phospholipase C/PKC-pathway are measured and compared to a reference sample. Specifically, the phosphorylation levels of at least one of LASP1, SKAP2, ZYX, RGS18, CALD1 and NAP1L1 and at least one of PLEK and ILK are measured. Preferably, the phosphorylation level of LASP1 and/or SKAP2 and the phosphorylation level of PLEK and/or ILK are measured. Even more preferred, the phosphorylation level of LASP1 and PLEK or the phosphorylation level of SKAP2 and PLEK are measured.

According to another specific embodiment of the method provided herein, the phosphorylation level of only one member of either the cAMP/PKA-pathway or the phospholipase C/PKC-pathway is measured and compared to a reference sample.

Specifically, the herein provided *in vitro* method of identifying abnormal platelet reactivity of a subject comprises the steps of:
i) providing a sample comprising the platelet proteome of said subject,
ii) measuring the phosphorylation level of LASP1, and
iii) comparing the phosphorylation level of LASP1 to the phosphorylation level of LASP1 in a reference sample,
wherein a difference in the phosphorylation level of at least 1.5 fold is indicative of abnormal platelet reactivity potential.

Specifically, in the case of diagnosis a decrease in the phosphorylation level of LASP1 of at least 1.5 fold, compared to a reference sample of a healthy subject or a pool of healthy subjects, corresponds to increased platelet reactivity potential, which indicates a vascular disease, whereas an increase in the phosphorylation level of LASP1 of at least 1.5 fold corresponds to decreased platelet reactivity potential which indicates a bleeding disorder.

Specifically, following treatment for vascular disease, an increase in the phosphorylation level of LASP1 of at least 1.5 fold, compared to an earlier sample of the subject, is indicative of treatment success, whereas following treatment for a bleeding disorder, a decrease in the phosphorylation level of LASP1 of at least 1.5 fold is indicative of treatment success.

Specifically, the herein provided *in vitro* method of identifying abnormal platelet reactivity of a subject comprises the steps of:
i) providing a sample comprising the platelet proteome of said subject,
ii) measuring the phosphorylation level of SKAP2, and
iii) comparing the phosphorylation level of SKAP2 to the phosphorylation level of SKAP2 in a reference sample,
wherein a difference in the phosphorylation level of at least 1.5 fold is indicative of abnormal platelet reactivity potential.

Specifically, in the case of diagnosis a decrease in the phosphorylation level of SKAP2 of at least 1.5 fold, compared to a reference sample of a healthy subject or a pool of healthy subjects, corresponds to increased platelet reactivity potential indicating a vascular disease, whereas an increase in the phosphorylation level of SKAP2 of at least 1.5 fold corresponds to decreased platelet reactivity potential indicating a bleeding disorder.

Specifically, following treatment for vascular disease an increase in the phosphorylation level of SKAP2 of at least 1.5 fold, compared to an earlier sample of the subject, is indicative of treatment success, whereas following treatment for a bleeding disorder, a decrease in the phosphorylation level of SKAP2 of at least 1.5 fold is indicative of treatment success.

Specifically, the herein provided *in vitro* method of identifying abnormal platelet reactivity of a subject comprises the steps of:
i) providing a sample comprising the platelet proteome of said subject,
ii) measuring the phosphorylation level of ZYX, and
iii) comparing the phosphorylation level of ZYX to the phosphorylation level of ZYX in a reference sample,
wherein a difference in the phosphorylation level of at least 1.5 fold is indicative of abnormal platelet reactivity potential.

Specifically, in the case of diagnosis a decrease in the phosphorylation level of ZYX of at least 1.5 fold, compared to a reference sample of a healthy subject or a pool of healthy subjects, corresponds to increased platelet reactivity potential indicating a vascular disease, whereas an increase in the phosphorylation level of ZYX of at least 1.5 fold corresponds to decreased platelet reactivity potential indicating a bleeding disorder.

Specifically, following treatment for vascular disease an increase in the phosphorylation level of ZYX of at least 1.5 fold, compared to an earlier sample of the subject, is indicative of treatment success, whereas following treatment for a bleeding disorder, a decrease in the phosphorylation level of ZYX of at least 1.5 fold is indicative of treatment success.

Specifically, the herein provided *in vitro* method of identifying abnormal platelet reactivity of a subject comprises the steps of:
i) providing a sample comprising the platelet proteome of said subject,
ii) measuring the phosphorylation level of RGS18, and
iii) comparing the phosphorylation level of RGS18 to the phosphorylation level of RGS18 in a reference sample,
wherein a difference in the phosphorylation level of at least 1.5 fold is indicative of abnormal platelet reactivity potential.

Specifically, in the case of diagnosis a decrease in the phosphorylation level of RGS18 of at least 1.5 fold, compared to a reference sample of a healthy subject or a pool of healthy subjects, corresponds to increased platelet reactivity potential indicating a vascular disease, whereas an increase in the phosphorylation level of RGS18 of at least 1.5 fold corresponds to decreased platelet reactivity potential indicating a bleeding disorder.

Specifically, following treatment for vascular disease an increase in the phosphorylation level of RGS18 of at least 1.5 fold, compared to an earlier sample of the subject, is indicative of treatment success, whereas following treatment for a bleeding disorder, a decrease in the phosphorylation level of RGS18 of at least 1.5 fold is indicative of treatment success.

Specifically, the herein provided *in vitro* method of identifying abnormal platelet reactivity of a subject comprises the steps of:
iv) providing a sample comprising the platelet proteome of said subject,
v) measuring the phosphorylation level of CALD1, and
vi) comparing the phosphorylation level of CALD1 to the phosphorylation level of CALD1 in a reference sample,
wherein a difference in the phosphorylation level of at least 1.5 fold is indicative of abnormal platelet reactivity potential.

Specifically, in the case of diagnosis an increase in the phosphorylation level of CALD1 of at least 1.5 fold, compared to a reference sample of a healthy subject or a pool of healthy subjects, corresponds to increased platelet reactivity potential indicating a vascular disease, whereas a decrease in the phosphorylation level of CALD1 of at least 1.5 fold corresponds to decreased platelet reactivity potential indicating a bleeding disorder.

Specifically, following treatment for vascular disease a decrease in the phosphorylation level of CALD1 of at least 1.5 fold, compared to an earlier sample of the subject, is indicative of treatment success, whereas following treatment for a bleeding disorder, an increase in the phosphorylation level of CALD1 of at least 1.5 fold is indicative of treatment success.

Specifically, the herein provided *in vitro* method of identifying abnormal platelet reactivity of a subject comprises the steps of:
i) providing a sample comprising the platelet proteome of said subject,
ii) measuring the phosphorylation level of NAP1L1, and
iii) comparing the phosphorylation level of NAP1L1 to the phosphorylation level of NAP1L1 in a reference sample,
wherein a difference in the phosphorylation level of at least 1.5 fold is indicative of abnormal platelet reactivity potential.

Specifically, in the case of diagnosis an increase in the phosphorylation level of NAP1L1 of at least 1.5 fold, compared to a reference sample of a healthy subject or a pool of healthy subjects, corresponds to increased platelet reactivity potential indicating a vascular disease, whereas a decrease in the phosphorylation level of NAP1L1 of at least 1.5 fold corresponds to decreased platelet reactivity potential indicating a bleeding disorder.

Specifically, following treatment for vascular disease a decrease in the phosphorylation level of NAP1L1 of at least 1.5 fold, compared to an earlier sample of the subject, is indicative of treatment success, whereas following treatment for a bleeding disorder, an increase in the phosphorylation level of NAP1L1 of at least 1.5 fold is indicative of treatment success.

Specifically, the herein provided *in vitro* method of identifying abnormal platelet reactivity of a subject comprises the steps of:
i) providing a sample comprising the platelet proteome of said subject,
ii) measuring the phosphorylation level of PLEK, and
iii) comparing the phosphorylation level of PLEK to the phosphorylation level of PLEK in a reference sample,
wherein a difference in the phosphorylation level of at least 1.5 fold is indicative of abnormal platelet reactivity potential.

Specifically, in the case of diagnosis an increase in the phosphorylation level of PLEK of at least 1.5 fold, compared to a reference sample of a healthy subject or a pool of healthy subjects, corresponds to increased platelet reactivity potential indicating a vascular disease, whereas a decrease in the phosphorylation level of PLEK of at least 1.5 fold corresponds to decreased platelet reactivity potential indicating a bleeding disorder.

Specifically, following treatment for vascular disease a decrease in the phosphorylation level of PLEK of at least 1.5 fold, compared to an earlier sample of the subject, is indicative of treatment success, whereas following treatment for a bleeding disorder, an increase in the phosphorylation level of PLEK of at least 1.5 fold is indicative of treatment success.

Specifically, the herein provided *in vitro* method of identifying abnormal platelet reactivity of a subject comprises the steps of:
iv) providing a sample comprising the platelet proteome of said subject,
v) measuring the phosphorylation level of ILK, and
vi) comparing the phosphorylation level of ILK to the phosphorylation level of ILK in a reference sample,
wherein a difference in the phosphorylation level of at least 1.5 fold is indicative of abnormal platelet reactivity potential.

Specifically, in the case of diagnosis a decrease in the phosphorylation level of ILK of at least 1.5 fold, compared to a reference sample of a healthy subject or a pool of healthy subjects, corresponds to increased platelet reactivity potential indicating a vascular disease, whereas an increase in the phosphorylation level of ILK of at least 1.5 fold corresponds to decreased platelet reactivity potential indicating a bleeding disorder.

Specifically, following treatment for vascular disease an increase in the phosphorylation level of ILK of at least 1.5 fold, compared to an earlier sample of the subject, is indicative of treatment success, whereas following treatment for a bleeding disorder, a decrease in the phosphorylation level of ILK of at least 1.5 fold is indicative of treatment success.

According to a preferred embodiment of the method provided herein, the reference sample is an earlier sample of the same subject or a sample of a healthy subject or of a pool of healthy subjects. Preferably, the reference sample is an earlier sample of the same subject for the assessment of treatment success. Specifically, an earlier sample can be at least 1 hour, 1 day, 1 week, 1 month or 1 year old or older. Preferably, for the diagnosis of platelet activation and/or inactivation disorders, the reference sample is a sample of a healthy subject or of a pool of healthy subjects.

According to a specific embodiment the sample and the reference sample can be any of whole blood, platelet rich plasma (PRP), platelet poor plasma (PPP), platelet enriched fraction, tissue, urine, serum, plasma, amniotic fluid, cerebrospinal fluid, placental cells or tissue, endothelial cells, leukocytes or monocytes. Specifically, the reference sample can also be any one or more of phosphorylated or unphosphorylated LASP1, SKAP2, ZYX, RGS18, CALD, NAP1L1, PLEK and ILK protein.

According to a specific embodiment of the method provided herein, the platelet proteome is derived from a sample by gel-filtration and subsequent precipitation under conditions avoiding further platelet activation.

According to a preferred embodiment, the platelet proteome is the non-secretory platelet proteome. Preferably, platelets are extracted from a sample by gel-filtration and gel-filtered platelets (GFP) are subsequently precipitated under conditions avoiding further platelet activation to yield the non-secretory platelet proteome. Preferably, the sample is platelet rich plasma (PRP) or whole blood, which is centrifuged to yield platelet rich plasma.

Preferably, a blood sample from a subject is drawn into sodium-citrate tubes which are centrifuged to obtain PRP. Optionally, platelets are then gently separated from plasma proteins by size-exclusion chromatography, yielding gel-filtered platelets (GFP). PRP or GFP are precipitated to obtain the whole platelet proteome or the non-secretory platelet proteome, respectively. Preferably, trichloroacetic acid and/or dithiotheitol (DTT) are used for precipitation. Specifically, all steps are performed under conditions avoiding (further) platelet activation.

According to a specific embodiment of the invention, an *in vitro* method for the diagnosis of a vascular disorder of a subject is provided, which *in vitro* method comprises the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample of a healthy subject or a pool of healthy subjects,
   i) wherein a decrease of at least 1.5 fold in the phosphorylation level of any one or more of LASP1, SKAP2, ZYX and RGS18 indicates increased platelet reactivity potential,
   ii) wherein an increase of at least 1.5 fold in the phosphorylation level of CALD1 and/or NAP1L1 indicates increased platelet reactivity potential, and
wherein increased platelet reactivity potential identifies the subject as being at risk of or having a vascular disorder.

According to a further specific embodiment of the invention, an *in vitro* method for the identification of treatment success of a subject having a vascular disorder is provided, comprising the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample, which is an earlier sample of said subject,
   i) wherein an increase of at least 1.5 fold in the phosphorylation level of any one or more of LASP1, SKAP2, ZYX and RGS18 indicates decreased platelet reactivity potential,
   ii) wherein a decrease of at least 1.5 fold in the phosphorylation level of CALD1 and/or NAP1L1 indicates decreased platelet reactivity potential, and wherein decreased platelet reactivity potential is indicative of treatment success.

According to a further specific embodiment of the invention, an *in vitro* method for the diagnosis of a vascular disorder of a subject is provided, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample of a healthy subject or a pool of healthy subjects,
   i) wherein an increase of at least 1.5 fold in the phosphorylation level of PLEK indicates increased platelet reactivity potential,
   ii) wherein a decrease of at least 1.5 fold in the phosphorylation level of ILK indicates increased platelet reactivity potential, and
wherein increased platelet reactivity potential identifies the subject as being at risk of or having a vascular disorder.

According to a further specific embodiment of the invention, an *in vitro* method for the identification of treatment success of a subject having a vascular disorder is provided, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample, which is an earlier sample of said subject,
   i) wherein a decrease of at least 1.5 fold in the phosphorylation level of PLEK indicates decreased platelet reactivity potential,
   ii) wherein an increase of at least 1.5 fold in the phosphorylation level of ILK indicates decreased platelet reactivity potential, and
wherein decreased platelet reactivity potential is indicative of treatment success.

According to yet a further specific embodiment of the invention, an *in vitro* method for the diagnosis of a bleeding disorder of a subject is provided, comprising the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample of a healthy subject or a pool of healthy subjects,
   i) wherein an increase of at least 1.5 fold in the phosphorylation level of any one or more of LASP1, SKAP2, ZYX and RGS18 indicates decreased platelet reactivity potential,
   ii) wherein a decrease of at least 1.5 fold in the phosphorylation level of CALD1 and/or NAP1L1 indicates decreased platelet reactivity potential, and
wherein decreased platelet reactivity potential identifies the subject as being at risk of or having a bleeding disorder.

According to a further specific embodiment of the invention, an *in vitro* method for the identification of treatment success of a subject having a bleeding disorder is provided, comprising the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample, which is an earlier sample of said subject,
   i) wherein a decrease of at least 1.5 fold in the phosphorylation level of any one or more of LASP1, SKAP2, ZYX and RGS18 indicates increased platelet reactivity potential,
   ii) wherein an increase of at least 1.5 fold in the phosphorylation level of CALD1 and/or NAP1L1 indicates increased platelet reactivity potential, and
wherein increased platelet reactivity potential is indicative of treatment success.

According to a further specific embodiment of the invention, an *in vitro* method for the diagnosis of a bleeding disorder of a subject is provided, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample of a healthy subject or a pool of healthy subjects,
   i) wherein a decrease of at least 1.5 fold in the phosphorylation level of PLEK indicates decreased platelet reactivity potential,
   ii) wherein an increase of at least 1.5 fold in the phosphorylation level of ILK indicates decreased platelet reactivity potential, and
wherein decreased platelet reactivity potential identifies the subject as being at risk of or having a bleeding disorder.

According to a further specific embodiment of the invention, an *in vitro* method for the identification of treatment success of a subject having a bleeding disorder is provided, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample, which is an earlier sample of said subject,
   i) wherein an increase of at least 1.5 fold in the phosphorylation level of PLEK indicates increased platelet reactivity potential,
   ii) wherein a decrease of at least 1.5 fold in the phosphorylation level of ILK indicates increased platelet reactivity potential, and
wherein increased platelet reactivity potential is indicative of treatment success.

According to a specific embodiment of the *in vitro* method provided herein, the phosphorylation level of the at least one member of the cAMP/PKA-pathway and/or the at least one member of the phospholipase C/PKC-pathway is detected using binding proteins specifically recognizing phosphorylated LASP1, SKAP2, ZYX, RGS18, CALD, NAP1L1, PLEK or ILK. Specifically, said binding proteins are selected from the group consisting of antibodies, antibody fragments and peptides. Antibody fragments can be any of a Fab, Fv, scFv, dAb, F(ab)2 or Fcab fragment, or any other possible binding entity, as long as it specifically binds the phosphorylated protein.

Upon platelet inactivation, e.g. following prostacyclin (PGI₂) treatment, ZYX is phosphorylated at Ser169, Thr469, Ser505 and Ser545. Preferably, the binding protein used in the *in vitro* method provided herein is an antibody or antibody fragment, which specifically binds ZYX phosphorylated at Ser169, Thr469, Ser505 and/or Ser545.

Upon platelet activation, e.g. following ADP or TRAP-6 treatment, PLEK is phosphorylated at Thr54 and Ser53. Preferably, the binding protein used in the *in vitro* method provided herein is an antibody or antibody fragment, which specifically binds PLEK phosphorylated at Thr54 and Ser53.

Specifically, the bleeding disorder is selected from the group consisting of hemophilia A, hemophilia B, factor I deficiency, factor II deficiency, factor V deficiency, factor VII deficiency, factor X deficiency, factor XI deficiency, factor XII deficiency, factor XIII deficiency and von Willebrand's disease.

Specifically, the vascular disease is selected from the group consisting of coronary artery disease, coronary atherosclerosis, myocardial infarction, stroke, thrombosis, coronary occlusion, hypertensive heart disease, hypertension, peripheral artery disease and diabetes, specifically type II diabetes.

According to a specific embodiment of the invention, the subject identified as being at risk of having or having a bleeding disorder is subjected to substitution treatment or administered clotting factor concentrate and wherein the subject identified as being at risk of having or having a vascular disease is administered an antiplatelet agent.

Specifically, the antiplatelet agent is selected from the group consisting of irreversible cyclooxygenase inhibitors such as aspirin and Triflusal, adenosine diphosphate (ADP) receptor inhibitors such as clopidogrel, prasugrel, ticagrelor and ticlopidine, phosphodiesterase inhibitors such as cilostazol, protease-activated receptor-1 (PAR-1) antagonists such as vorapaxar, glycoprotein IIB/IIIA inhibitors such as abciximab, eptifibatide and tirofiban, adenosine reuptake inhibitors such as dipyridamole, thromboxane inhibitors, thromboxane synthase inhibitors and thromboxane receptor antagonists such as terutroban.

According to a further specific embodiment of the invention, a kit of parts is provided, which comprises
a) capturing reagents, preferably antibodies, capable of capturing at least one of phosphorylated and/or unphosphorylated LASP1, SKAP2, ZYX, RGS18, CALD, NAP1L1, PLEK and ILK, and
b) optionally, a reference sample, and
c) detection reagents for the detection of the phosphorylated and/or unphosphorylated proteins of a), and
an instruction leaflet comprising information on the correlation of increased or decreased phosphorylation levels of at least one of LASP1, SKAP2, ZYX, RGS18, CALD1, NAP1L1, PLEK and ILK with increased or decreased platelet reactivity potential corresponding to vascular disease and bleeding disorder.

According to a further specific embodiment of the invention, a kit of parts of a panel of platelet proteins is provided which comprises
a) capturing reagents, preferably antibodies, capable of capturing two or more of, preferably all of, phosphorylated and/or unphosphorylated LASP1, SKAP2, ZYX, RGS18, CALD, NAP1L1, PLEK and ILK, and
b) optionally, a reference sample, and
c) detection reagents for the detection of the phosphorylated and/or unphosphorylated proteins of a), and
an instruction leaflet comprising information on the correlation of increased or decreased phosphorylation levels of two or more of, preferably all of LASP1, SKAP2, ZYX, RGS18, CALD, NAP1L1, PLEK and ILK with increased or decreased platelet reactivity potential corresponding to vascular disease and bleeding disorder.

Preferably, capturing reagents are binding proteins immobilized to a solid phase and which are capable of specifically recognizing and binding phosphorylated and/or unphosphorylated LASP1, SKAP2, ZYX, RGS18, CALD, NAP1L1, PLEK or ILK. Specifically, said binding protein, also called primary binding protein, is selected from the group consisting of antibodies, antibody fragments and peptides. Antibody fragments can be any of a Fab, Fv, scFv, dAb, F(ab)2 or Fcab fragment, or any other possible binding entity, as long as it specifically binds the phosphorylated and/or unphosphorylated protein.

Preferably, detection reagents for the detection of the phosphorylated and/or unphosphorylated proteins are antibodies specifically recognizing either captured proteins or primary binding proteins and which comprise a detectable tag such as fluorogenic, chromogenic or chemiluminescent tags. Preferably, the detection reagent is a fluorescently labelled antibody or an enzymatically labelled antibody. Preferably, luciferase, alkaline phosphatase or horseradish peroxidase are used as enzymatic labels of proteins.

According to a specific embodiment of the kits provided herein, the detection reagent is an enzymatically labelled antibody and the kit further comprises enzymatic substrates such as luciferin, coelenterazine, ABTS (the diammonium salt of 2,2'-azino-di-(3-ethylbenzthiazoline-6-sulfonic acid)), PNPP (p-nitrophenyl phosphate), OPD (peroxidase ortho-phenylenediamine) or TMB (3,3',5,5'-tetramethylbenzidine).

Preferably, said detection reagents are binding proteins specifically recognizing phosphorylated LASP1, SKAP2, ZYX, RGS18, CALD, NAP1L1, PLEK or ILK. Specifically, said binding protein is selected from the group consisting of antibodies, antibody fragments and peptides. Antibody fragments can be any of a Fab, Fv, scFv, dAb, F(ab)2 or Fcab fragment, or any other possible binding entity, as long as it specifically binds the phosphorylated protein.

Preferably, the capturing and/or detection reagent used in the method and in the kit provided herein is an antibody or antibody fragment, which specifically binds ZYX phosphorylated at Ser169, Thr469, Ser505 and/or Ser545.

Preferably, the capturing and/or detection reagent used in the method and in the kit provided herein is an antibody or antibody fragment, which specifically binds PLEK phosphorylated at Thr54 and Ser53.

### FIGURES

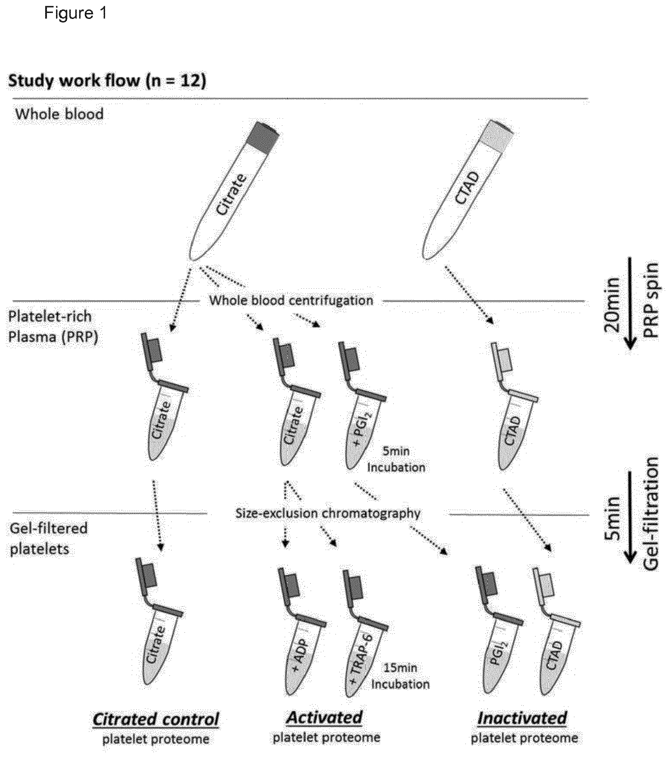

**Figure 1****:** Study work flow. Whole blood was drawn into citrate and CTAD blood tubes from 12 healthy volunteers. Blood was then centrifuged at 120 xg for 20 minutes at room temperature to obtain platelet-rich plasma (PRP). For platelet inactivation, citrated PRP was incubated with PGI₂ (0.4 µM final concentration) for 5 minutes prior to gel-filtration (size-exclusion chromatography), while CTAD-PRP was left untreated. For platelet activation, gel-filtered citrated platelets were incubated with 5 µM ADP and 15 µM TRAP-6 for 15 minutes. Citrated gel-filtered platelets were used as control for activated and inactivated platelet proteome analysis. n = 6 (PGI₂, CTAD), n= 6 (ADP, TRAP-6).

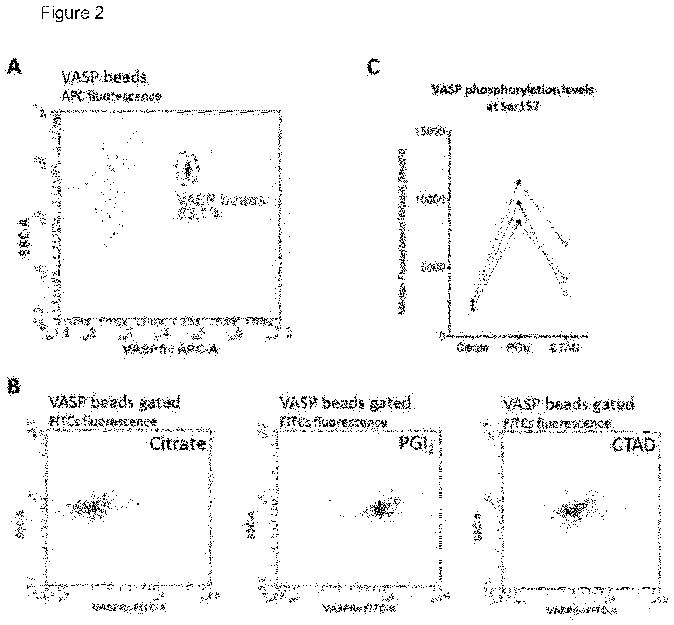

**Figure 2****:** Validation of inactivation-related VASP phosphorylation profiles by flow cytometry after PGI₂ and CTAD treatment. To validate our 2D-DIGE findings of a significant increase of VASP phosphorylation in PGI₂, but not in CTAD-treated platelets, we quantified the VASP-Ser157 phosphorylation by flow cytometry. (A) VASP-protein was detected in perforated gel-filtered platelets of citrate, PGI₂ and CTAD via allophycocyanin (APC-) fluorescence-labeled beads (exemplary plot of one measurement). (B) Gated VASP beads were then quantified via fluorescein isothiocyanate-(FITC) fluorescence-labeled antibodies at phosphorylation site VASP-Ser157 in the three treatment groups citrate, PGI₂ and CTAD. (C) Signals are quantitatively depicted as median fluorescence intensity (median FI) in three individuals and were compared by a Mann-Whitney-U test. The analysis showed a +4.21-FC median fluorescence intensity in PGI₂ treated platelets (p= 0.03) compared to +1.98-FC in CTAD (p = 0.44).

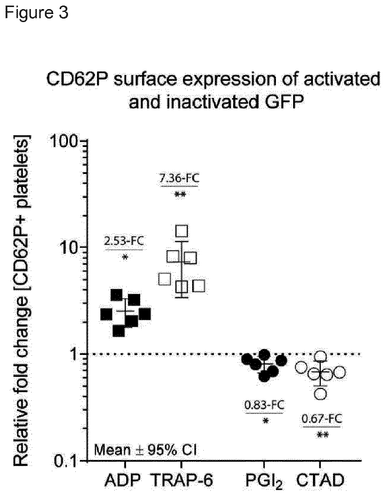

**Figure 3****:** CD62P surface expression of activated and inactivated gel-filtered platelets. Activation status of platelets subjected to 2D-DIGE was determined by quantifying percentage of platelet CD62P- (P-selectin) surface expression using flow cytometry. Gel-filtered platelets treated with ADP, TRAP-6, PGI₂ or CTAD were incubated for 15 minutes with a phycoerythrin (PE)-labeled antibody against CD62P. The CD62P-positive platelets of each individual are depicted as relative fold changes to untreated citrated gel-filtered platelets (dashed line at y = 1). For each comparison, the average fold change (FC) and the significance level are given in the graph. The null hypothesis was tested by a one-sample t-test with an α-level set at 0.05. n = 6 (PGI2, CTAD), n = 6 (ADP, TRAP-6). * p < 0.05 ** p < 0.01.

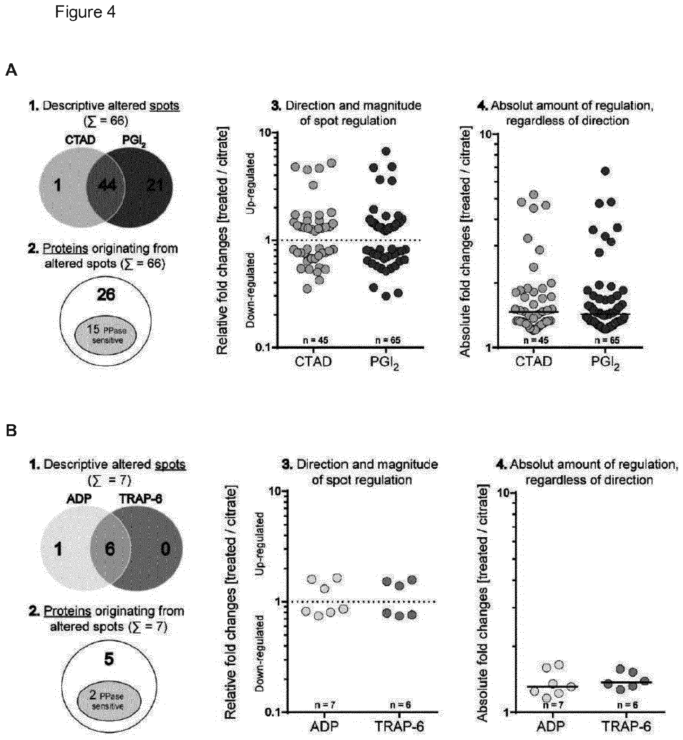

**Figure 4****:** Quantitative and qualitative platelet proteome changes in response to **(A) inactivation** (CTAD/ PGI₂) and **(B) activation** (ADP/ TRAP-6) compared to baseline (citrate). In sum, 66 spots for inactivation and 7 spots for activation were significantly altered. These spots were deduced from a total of 5,906 detected protein spots by applying the following criteria: [(a) protein spots matched > 90% of all 2D-DIGE gels, (b) protein abundance changes > 20% between treatment group (ADP, TRAP-6, CTAD or PGI₂) and citrated control and (c) FDR-corrected ANOVA [p-value<0.05]. (1) Absolute numbers of separately and commonly altered protein spots upon inactivation (CTAD , PGI₂ ; Σ = 66) and activation (ADP , TRAP-6 ; Σ = 7). (2) Altered protein spots upon inactivation (Σ = 66) and activation (Σ = 7), that originated from distinct proteins: in sum, 31 proteins were identified (26-inactivation; 5-activation) of which 17 proteins were sensitive to phosphatase treatment (15-inactivation; 2-activation) (3) The direction and magnitude of regulation is given for commonly and uniquely altered protein spots of each subgroup: CTAD = 45 (1 unique /44 common), PGI₂ = 65 (21/44), ADP = 7 (1/6) and TRAP-6 = 6 (0/6). Relative fold changes [treated vs. citrate] are depicted and compared to the respective control (dashed line y = 1) on a logarithmic scale. (4) The absolute amount (|x|) of altered protein spots, regardless of direction, is represented as positive fold changes [treated vs. citrate] compared to the respective control with its corresponding medians. The median abundance difference was determined by a Mann-Whitney-U test with α-level set at p < 0.05.

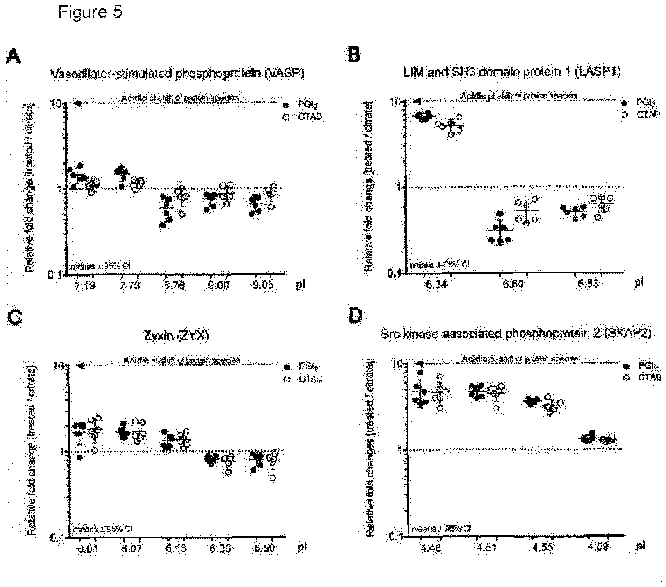

**Figure 5****:** Platelet inactivation (by PGI₂ or CTAD) induces shifts of protein spots to a more acidic pI. Significantly changed protein species, belonging to (A) VASP, (B) LASP1, (C) ZYX and (D) SKAP2 are depicted along their pI ranges. Values of each protein spots are given as relative fold changes between treated gel-filtered platelets (PGI₂ ●, CTAD ○) and untreated citrated control (dashed line y = 1) with their means and 95% confidence interval (n = 6 in each subgroup). Statistical significance was set at p < 0.05 and was determined by post-hoc testing between subgroups (PGI₂, CTAD) and untreated controls. PGI₂- and CTAD-dependent protein profiles of VASP were also validated by flow cytometry (Figure 2).

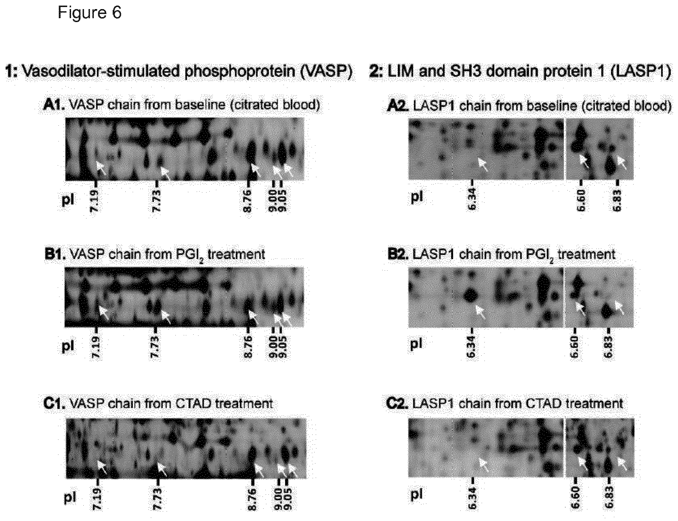

**Figure 6****:** Detection of phosphorylated protein species of VASP and LASP1 by A-phosphatase treatment. Spot abundances of (1) five VASP spots and (2) three LASP1 spots are shown in (A) citrate-, (B) PGI₂-, and (C) PGI₂+λ-phosphatase treated platelets along their pI ranges. (A) Platelet protein extracts from citrated blood constitute baseline settings. (B) PGI₂-treatment shifts protein spots towards the acidic pH-range with a concomitant reduction of the corresponding alkaline spot abundances. (C) Λ-phosphatase treatment removes the phosphorylation of protein species thereby shifting its isoelectric point (pI) towards the alkaline direction. Images (A-C) constitute a representative picture from one individual. The gel-sections were obtained from scanned 2D-DIGE gels and represent the respective protein area. The LASP1 images (2) overlap (vertical solid line) between pH 6-7 due to its wide-spreading across the pI-range (pH 6-7). Identified protein spots are marked with white arrows and their corresponding pI.

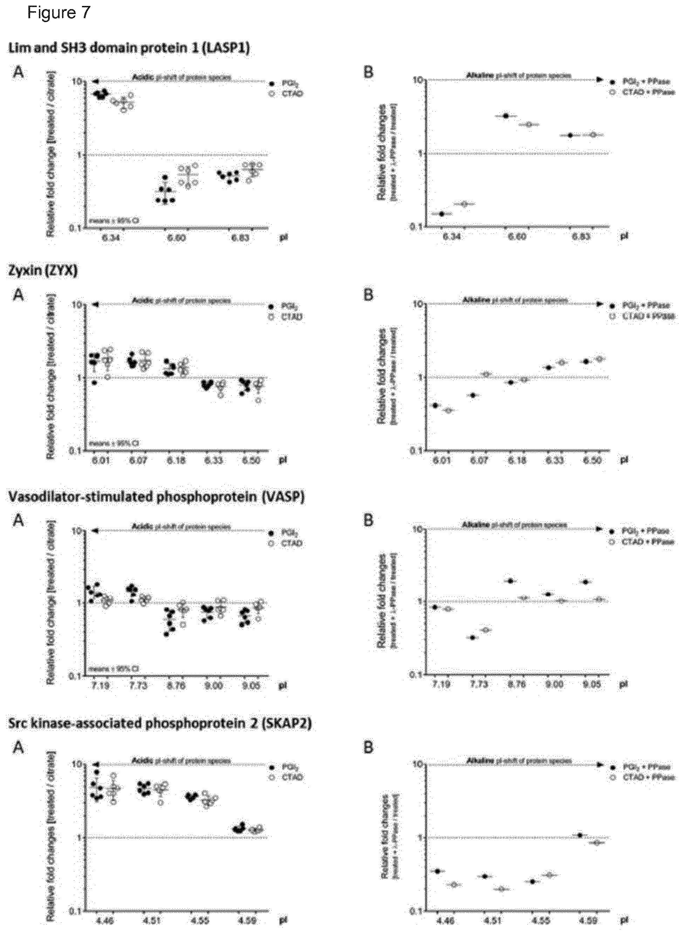

**Figure 7****:** Detection of phosphorylated protein species of the known cAMP/PKA targets LASP1, ZYX, VASP and potential cAMP/PKA target SKAP2 by A-phosphatase treatment. (A) Upon inactivation (PGI2, CTAD) significantly changed protein species of known and potential cAMP/PKA targets LASP1, ZYX, VASP and SKAP2 are depicted along their pI ranges. Values of each protein species are given as relative fold changes between treated gel-filtered platelets (PGI2, CTAD) and untreated citrated control (dashed line y = 1) with their means and 95% confidence interval (CI). For LASP1, ZYX, VASP and SKAP2 an acidic pI shift of protein species upon inactivation was detectable. (B) To determine if a possible post-translational phosphorylation of protein species is responsible for this shift, PGI2- and CTAD- treated gel-filtered platelet were incubated with A-phosphatase and compared to PGI2- and CTAD-treated gel-filtered platelets, respectively. The A-phosphatase removes the phosphorylation from serine, threonine and tyrosine residues, thereby shifting its isoelectric point (pI) to the alkaline direction, displaying a mirror-inverted picture to the abundance changes of protein species seen in (A).

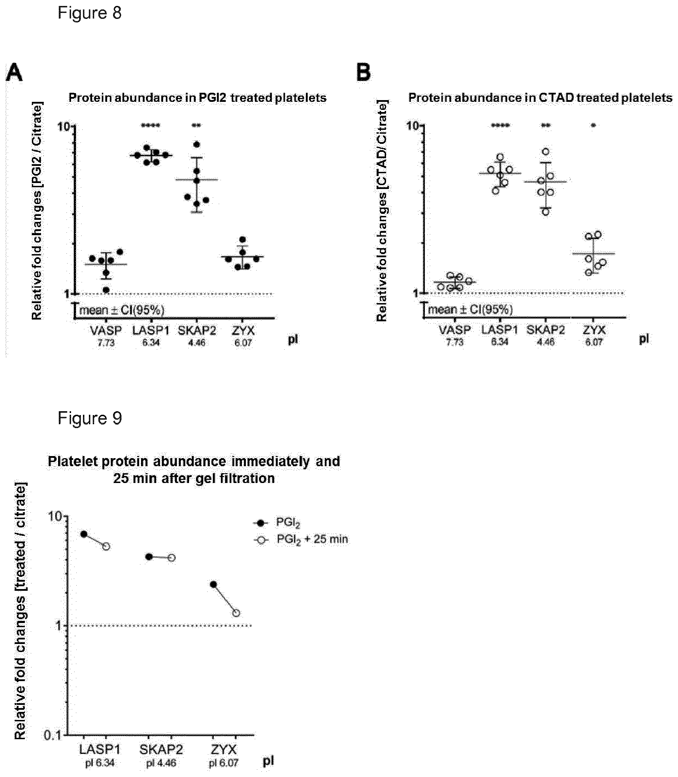

**Figure 8****:** Phosphorylation induced by inactivation causes stronger protein abundance changes in LASP1, SKAP2 than in VASP. The most acidic protein spot abundances of VASP, LASP1, SKAP2 and ZYX are depicted as fold changes between treated gel-filtered platelets after (A) PGI₂ and (B) CTAD treatment with their means, 95% confidence interval (CI) and isoelectric point (n=6 in every group). Statistical significance was set at p < 0.05 and determined by a repeated ANOVA with single post-hoc contrast compared to VASP. Statistical significance is marked and represents the respective acidic protein abundance to VASP abundance. * p < 0.05 ** p < 0.01 *** p < 0.001 **** p < 0.0001.
**Figure 9****:** Validation of phosphorylation stability of LASP1, SKAP2 and ZYX protein species. Phosphorylation stability was determined in four known and potential cAMP/PKA targets. Protein species with the highest abundance in phosphorylation were selected. Here, PRP was incubated with 0.4 µM PGI₂ prior to gel-filtration. Obtained gel-filtered platelets were either precipitated immediately or left for another 25 minutes at room temperature before precipitation. Protein abundances were determined by 2D-DIGE analysis and normalized by the same internal standard as used in all previous experiments. Fold changes between spot abundances and citrated untreated control are depicted (n = 1).

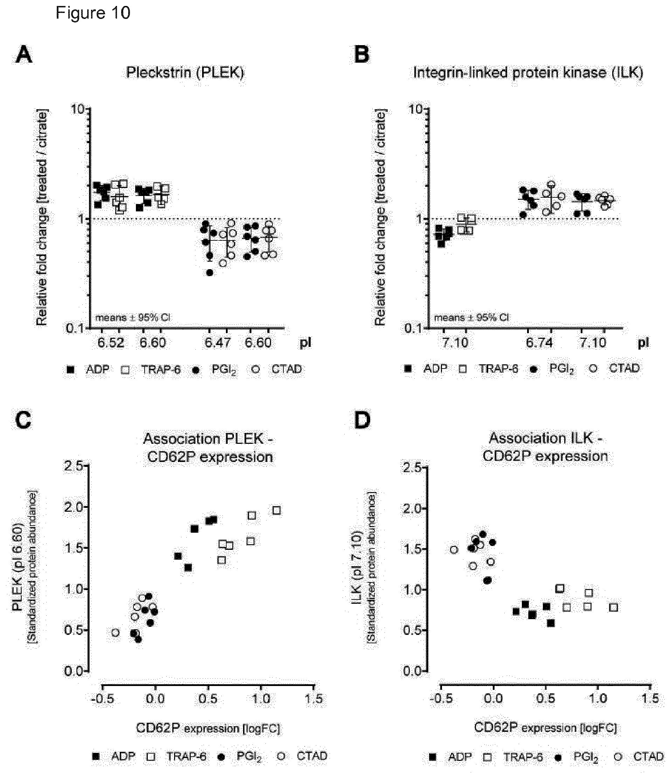

**Figure 10****:** Both platelet inactivation and activation regulate PLEK and ILK protein abundance. The protein abundance changes of (A) PLEK and (B) ILK are depicted as fold changes between treated gel-filtered platelets (ADP , TRAP-6 , PGI₂ or CTAD) and untreated citrated control (dashed line y = 1) with their means and 95% confidence interval (CI). Since (C) PLEK (pI 6.60), (D) ILK (pI 7.10) and platelet CD62P expression are affected by activation and inactivation, the association between protein levels and functional parameters was assessed showing a scatter dot plot of the relation between protein levels [relative fold changes] and the logarithmic platelet CD62P surface expression [relative fold change]. n = 6 (PGI₂, CTAD), n= 6 (ADP, TRAP-6).

### DETAILED DESCRIPTION

Specific terms as used throughout the specification have the following meaning.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/- 10% of the given value.

Platelets are the primary players in both thrombosis and hemostasis. Increased platelet reactivity may result in thrombosis and other vascular diseases, whereas decreased platelet reactivity may indicate a bleeding disorder. Investigating the platelet proteome after *in vitro* activation and inactivation yielded sensitive and robust biomarkers for the diagnosis of vascular diseases and bleeding disorders and to monitor platelet activation potential and reactivity in patients receiving treatment for vascular diseases or bleeding disorders.

Platelets are anucleate and were previously considered to be simple cytoplasmic fragments of megakaryocytes. It is now known that platelets house an array of molecules, including proteins, RNA, and the subcellular machinery of de novo protein synthesis. Platelets can synthesize new proteins, such as the integrin alpha3 (glycoprotein-IIIa) protein and the fibrinogen receptor and they can mediate posttranslational modifications.

Platelets are preserved in a sensitive balance between upholding adequate blood-flow and promoting blood clotting in the case of bleeding. Upon vessel injury, platelets instantly become activated, leading to granule release, aggregation, and plug formation. Under balanced physiological conditions, endothelial cells produce prostacyclin (PGI₂) and nitric oxide, thereby keeping platelets silenced. Dysregulation and inappropriate activation of platelets underpin a wide range of important diseases, including stroke and myocardial infarction, whereas impairment of platelet function results in bleeding disorders.

The term "abnormal platelet reactivity potential" thus refers to an increase or decrease of the platelet reactivity potential compared to physiological platelet reactivity potential. Under physiological conditions platelets are silenced and are activated in the event of hemostasis, forming blood clots to stop the bleeding at the site of an interrupted epithelium. Major *in vivo* triggers for activation include binding of sub-endothelial collagen as well as other platelet agonists, like adenosine-diphosphate (ADP) and thrombin, to their cognate receptors. Under balanced physiological conditions, endothelial cells produce prostacyclin (PGI₂) and nitric oxide, thereby keeping platelets silenced. Both mediators induce the synthesis of cyclic nucleotides (cAMP, cGMP), which activate protein kinases A and G (PKA, PKG) in platelets. These serine/threonine kinases phosphorylate particular proteins to counteract the activation of platelets. Platelet activation and thrombus formation can, therefore, be regarded as the predominance of either pro-thrombotic factors or a reduction of endogenous inhibitors. An impairment of platelet-inactivation mechanisms, or resistance to them, may result in hyper-reactive platelets and spontaneous generation of platelet-dependent thrombosis.

Identifying abnormal platelet reactivity potential using sensitive and robust biomarkers is thus essential for the following:
1. Identification of subjects at increased risk of having or having a vascular disease or bleeding disorder.
2. Monitoring response to antiplatelet agents, substitution treatment or clotting factor concentrate, to assist in personalizing treatment regimens, specifically antiplatelet therapy.
3. Assessment of pharmacodynamic effects to optimally time surgical procedures to reduce wait time and bleeding.

The term "cAMP/PKA-pathway" refers to a signaling cascade comprising cAMP and PKA as main players in a complex system of interacting proteins. Cyclic AMP (cAMP) and cAMP-dependent protein kinase (PKA) are important signaling molecules for the regulation of platelet function, such as adhesion, aggregation, and secretion. Elevation of intracellular cAMP, which induces the activation of PKA, results in the inhibition of platelet function. Thus, tight control of the intracellular cAMP/PKA signaling pathway has great implications for platelet-dependent hemostasis and effective cardiovascular therapy.

Cyclic AMP-dependent protein kinase (protein kinase A, PKA), a serine/threonine kinase highly expressed in human platelets, is an integral constituent of a kinase cascade that links a number of extracellular signals to a variety of cellular functions. It has been generally accepted that activation of PKA by increased cyclic AMP (cAMP) concentration results in the inhibition of platelet activation, however, the mechanism and the exact constitution of the pathway and its members are still unclear.

Members of the cAMP/PKA-pathway are not only cAMP and PKA but also other signaling molecules, substrates and proteins which are affected, specifically phosphorylated, downstream of cAMP and/or PKA. Further, members of the cAMP/PKA-pathway are also signaling molecules and proteins upstream of cAMP and/or PKA. Upstream regulators of cAMP or PKA can lead to activation or inhibition of cAMP and/or PKA, either directly or indirectly. Specifically, members of the cAMP/PKA-pathway are, among others, LASP1, SKAP2, ZYX, RGS18, CALD1, NAP1L1 and VASP.

For example, the cAMP/PKA-pathway member VASP is phosphorylated by PKA, which in turn is activated by cAMP. VASP is therefore downstream of cAMP and PKA. Phosphorylation of VASP by PKA might be one of the mechanisms in cAMP/PKA-mediated inhibition of platelet adhesion.

As another example, the cAMP/PKA-pathway member **RGS18** is also phosphorylated by PKA and therefore downstream of cAMP and PKA. cAMP-mediated phosphorylation of RGS18 on Ser126 causes it to dissociate from 14-3-3y. It is known that free RGS18 levels rise when platelets are rendered resistant to activation by exposure to prostaglandin I₂ (PGI₂) or forskolin, both of which increase platelet cyclic adenosine monophosphate (cAMP) levels.

In platelets treated with CTAD or prostacyclin (PGI₂) the phosphorylation level of RGS18 is significantly altered, compared to untreated platelets. Specifically, following CTAD or prostacyclin (PGI₂) mediated inactivation the phosphorylation level of RGS18 is increased by at least 1.5 fold in inactivated platelets. Conversely, an at least 1.5 fold decreased phosphorylation level of RGS18 compared to the phosphorylation level of RGS18 of inactivated platelets is indicative of increased platelet reactivity.

LIM and SH3 domain protein 1 **(LASP1)** is a member of the LIM protein subfamily which is characterized by a LIM motif and a domain of Src homology region 3. It is known to function as an actin-binding protein and it is known to interact with Zyxin. In platelets treated with CTAD or prostacyclin (PGI₂) the phosphorylation level of LASP1 is significantly altered, compared to untreated platelets. Specifically, following CTAD or prostacyclin (PGI₂) mediated inactivation the phosphorylation level of LASP1 is increased by at least 1.5 fold in inactivated platelets. Conversely, an at least 1.5 fold decreased phosphorylation level of LASP1 compared to the phosphorylation level of LASP1 of inactivated platelets is indicative of increased platelet reactivity.

Src kinase associated phosphoprotein 2 **(SKAP2)** shares homology with Src kinase-associated phosphoprotein 1, and is a substrate of Src family kinases. It contains an amino terminal coiled-coil domain for self-dimerization, a plecskstrin homology (PH) domain required for interactions with lipids at the membrane, and a Src homology (SH3) domain at the carboxy terminus. Some reports indicate that this protein inhibits actin polymerization through interactions with actin assembly factors. In platelets treated with CTAD or prostacyclin (PGI₂) the phosphorylation level of SKAP2 is significantly altered, compared to untreated platelets. Specifically, following CTAD or prostacyclin (PGI₂) mediated inactivation the phosphorylation level of SKAP2 is increased by at least 1.5 fold in inactivated platelets. Conversely, an at least 1.5 fold decreased phosphorylation level of SKAP2 compared to the phosphorylation level of SKAP2 of inactivated platelets is indicative of increased platelet reactivity.

Zyxin (**ZYX**) is a zinc-binding phosphoprotein that concentrates at focal adhesions and along the actin cytoskeleton. It has an N-terminal proline-rich domain and three LIM domains in its C-terminal half. The proline-rich domain may interact with SH3 domains of proteins involved in signal transduction pathways while the LIM domains are likely involved in protein-protein binding. It has been shown to interact with VASP and LASP1. In platelets treated with CTAD or prostacyclin (PGI₂) the phosphorylation level of ZYX is significantly altered, compared to untreated platelets. Specifically, following CTAD or prostacyclin (PGI₂) mediated inactivation the phosphorylation level of ZYX is increased by at least 1.5 fold in inactivated platelets. Conversely, an at least 1.5 fold decreased phosphorylation level of ZYX compared to the phosphorylation level of ZYX of inactivated platelets is indicative of increased platelet reactivity.

Caldesmon 1 **(CALD1)** is a calmodulin- and actin-binding protein. Its conserved domain possesses the binding activities to Ca²⁺-calmodulin, actin, tropomyosin, myosin, and phospholipids. In platelets treated with CTAD or prostacyclin (PGI2) the phosphorylation level of CALD1 is significantly altered, compared to untreated platelets. Specifically, following CTAD or prostacyclin (PGI₂) mediated inactivation the phosphorylation level of CALD1 is decreased by at least 1.5 fold in inactivated platelets. Conversely, an at least 1.5 fold increased phosphorylation level of CALD1 compared to the phosphorylation level of CALD1 of inactivated platelets is indicative of increased platelet reactivity.

Nucleosome assembly protein 1-like 1 **(NAP1L1)** is a member of the nucleosome assembly protein (NAP) family. It is known to participate in DNA replication and may play a role in modulating chromatin formation and contribute to the regulation of cell proliferation. In platelets treated with CTAD or prostacyclin (PGI₂) the phosphorylation level of NAP1L1 is significantly altered, compared to untreated platelets. Specifically, following CTAD or prostacyclin (PGI₂) mediated inactivation the phosphorylation level of NAP1L1 is decreased by at least 1.5 fold in inactivated platelets. Conversely, an at least 1.5 fold increased phosphorylation level of NAP1L1 compared to the phosphorylation level of NAP1L1 of inactivated platelets is indicative of increased platelet reactivity.

The terms "phospholipase C/protein kinase C (PKC)-pathway" and "phospholipase C/PKC-pathway" as used herein refer to a signaling cascade comprising phospholipase C and PKC as main players in a complex system of interacting proteins. The phospholipase/PKC-pathway is an essential signaling mediator in platelet activation and aggregation. Stimulation of proteinase-activated receptors 1 and 4 in human platelets induces inside-out signaling through the activation of phospholipase C (PLC). Furthermore, collagen, the most abundant protein of the extracellular matrix, promotes the adhesion and activation of platelets through its binding to platelet integrin α₂β₁ and GPVI. Binding of collagen to GPVI stimulates a non-receptor protein tyrosine kinase that phosphorylates and thereby activates PLC. Activated PLC hydrolyzes phosphatidylinositol 4,5-bisphosphate, generating the second messengers diacylglycerol (DAG) and inositol 1,4,5-triphosphate. Inositol 1,4,5-triphosphate, in turn, mediates the release of Ca²⁺ from intracellular stores, whereas DAG activates PKCs.

Members of the phospholipase/PKC-pathway are not only phospholipase C and PKC but also other signaling molecules, substrates and proteins which are affected, specifically phosphorylated, downstream of phospholipase C and/or PKC. Further, members of the phospholipase/PKC-pathway are also signaling molecules and proteins upstream of phospholipase C and/or PKC. Upstream regulators of phospholipase C or PKC can lead to activation or inhibition of phospholipase C and/or PKC, either directly or indirectly. Specifically, members of the phospholipase/PKC-pathway are, among others, PLEK and ILK.

Pleckstrin **(PLEK)** is a major PKC substrate in platelets and a member of the phospholipase C/PKC-pathway. Pleckstrin accounts for 1% of the total protein in platelets, but it is best known for containing the 2 prototypic pleckstrin homology, or PH, domains. It is known that pleckstrin regulates the fusion of granules to the cell membrane and is an essential component of PKC-mediated exocytosis. In platelets activated with ADP or TRAP-6 the phosphorylation level of PLEK is significantly altered, compared to untreated platelets. Specifically, following ADP or TRAP-6 mediated activation the phosphorylation level of PLEK is increased by at least 1.5 fold in activated platelets. Therefore, an at least 1.5 fold increased phosphorylation level of PLEK compared to the phosphorylation level of PLEK of inactivated platelets is indicative of increased platelet reactivity.

Integrin-linked protein kinase (**ILK**) is an enzyme involved with integrin-mediated signal transduction. In platelets ILK associates with β1- and β3-containing integrins, which are of paramount importance for the function of platelets. In platelets activated with ADP or TRAP-6 the phosphorylation level of ILK is significantly altered, compared to untreated platelets. Specifically, following ADP or TRAP-6 mediated activation ILK is dephosphorylated at pI 7.10 and following CTAD or PGI₂ mediated inactivation ILK is phosphorylated at pI 7.10. Therefore, an at least 1.5 fold decreased phosphorylation level of ILK compared to the phosphorylation level of ILK of inactivated platelets is indicative of increased platelet reactivity.

The term "subject" or "test subject" refers to humans and non-human mammals, such as dogs, cats, horses, cattle, pigs, goats, sheep, camels, guinea pigs, mice, rats and rabbits and non-human primates, e.g. monkeys such as cynomolgous or rhesus monkeys or chimpanzees subjected to the *in vitro* method provided herein. The subject may be a human or a non-human. The subject may be a subject at risk of having a vascular disease or bleeding disorder or a subject already having a vascular disease or bleeding disorder.

The term "sample" refers to a sample or isolate which comprises the platelet proteome of the subject. The sample can be blood, platelet rich plasma (PRP), platelet poor plasma (PPP), platelet enriched fraction, tissue, urine, serum, plasma, amniotic fluid, cerebrospinal fluid, placental cells or tissue, endothelial cells, leukocytes or monocytes. It can be used directly as obtained from a subject or can be pre-treated such as by filtration, gel-filtration, precipitation, extraction, concentration, centrifugation, inactivation of interfering components, or addition of reagents to prepare the sample for the method as provided herein. Importantly, the sample is treated under conditions avoiding further platelet activation.

The term "reference sample" refers to an earlier sample of the test subject or to a sample of a healthy subject or of a pool of healthy subjects. The reference sample can be blood, platelet rich plasma (PRP), platelet poor plasma (PPP), platelet enriched fraction, tissue, urine, serum, plasma, amniotic fluid, cerebrospinal fluid, placental cells or tissue, endothelial cells, leukocytes, monocytes or one or more of phosphorylated or unphosphorylated LASP1, SKAP2, ZYX, RGS18, CALD, NAP1L1, PLEK and ILK protein. Specifically, for the diagnosis of vascular disease or bleeding disorders, the reference sample is a sample of a healthy subject or a pool of healthy subjects. Healthy subjects are human or non-human mammalian subjects who are not at risk of having or do not have a vascular disease or bleeding disorder. Specifically, for the identification of treatment success, the reference sample is an earlier sample of the test subject. Earlier samples of test subjects are usually taken before treatment is started, to determine treatment success, or during treatment, to monitor treatment success. Earlier samples can be taken regularly during the course of a treatment, specifically at regular intervals between administrations of individual treatment dosages.

The term "platelet proteome" as used herein refers to the pool of proteins expressed at a given time and circumstance in the platelet or secreted by the platelet. Over 1100 platelet proteins have been identified using proteomic techniques. Various subproteomes have been characterized, including platelet releasates (the "secretome"), alpha and dense granules, membrane and cytoskeletal proteins, platelet-derived microparticles, and the platelet "phosphoproteome". Proteomic data about platelets have become increasingly available in integrated databases.

The non-secretory platelet proteome comprises the pool of proteins expressed at any given time and circumstance in the platelet.

Proteomic experiments begin with a protein mixture which is digested to a peptide mixture, either in a gel or in solution. Two-dimensional electrophoresis (2-DE) has been available since the 1970s, and was used in the 1990s to construct early maps of platelet proteins. Newer gel-based methods include differential in-gel electrophoresis (DIGE), which allows for quantitation and comparison of samples from different proteomic states. In DIGE, samples are differentially labeled with fluorescent dyes and simultaneously analyzed in a single gel. Alternatively, protein spots may be excised from a gel, digested, and analyzed by mass spectrometry (MS). Mass spectrometry (MS) has the capacity to identify proteins in a high throughput manner, using bioinformatics approaches linked to protein sequence databases. The two main mass-spectrometric options are electrospray ionization (ESI) and matrix-assisted laser desorption/ionization (MALDI). Both MALDI and a related method, surface-enhanced laser desorption/ionization (SELDI), employ a matrix to ionize proteins; SELDI uses protein chips with chromatographic surfaces. Isotope affinity tagging techniques help to increase the sensitivity of detection of smaller peptide fragments and provide quantitative information about protein concentrations. Isotope coded affinity tags (ICAT) selectively label cysteine residues of peptide fragments following tryptic digest of the protein sample; this technique increases the depth of protein coverage but is limited by selective labeling. Isotope tags for relative and absolute quantitation (iTRAQ) label every tryptic fragment, thus allowing for measurement of absolute changes in protein composition.

Further alternatives to DIGE include gel-free methods which can detect proteins not well represented by 2-DE such as transmembrane and basic proteins and use in-solution proteolytic digestion. Multidimensional protein identification technology (MudPIT) employs ion-exchange and reverse-phase liquid chromatography (LC) for peptide separation.

According to a specific embodiment, an *in vitro* method is provided for the diagnosis of a subject suspected of having or being at risk of having a vascular disease or bleeding disorder. Accordingly, a sample comprising the platelet proteome of a subject is provided and the phosphorylation level of at least one member of the cAMP/PKA-pathway and/or at least one member of the phospholipase C/PKC-pathway is compared to the phosphorylation level of the corresponding pathway member of a reference sample. Specifically, said reference sample is a sample comprising the platelet proteome of a healthy subject or a pool of healthy subjects. A difference of at least 1.5 fold in the phosphorylation level of the at least one member of the cAMP/PKA-pathway and/or the phospholipase C/PKC-pathway indicates that the subject has or is at risk of having a vascular disease or bleeding disorder.

The term "risk of having a vascular disease" and the term "risk of having a bleeding disorder" as used herein means an evaluation of factors including biomarkers, to predict the risk of occurrence of vascular disease or bleeding disorder including increased probability of disease/disorder onset, disease/disorder progression and occurrence/severity of clinical symptoms associated with vascular disease or bleeding disorder.

According to a specific embodiment an *in vitro* method is provided for the identification of treatment success and monitoring of treatment success of a subject having a vascular disease or bleeding disorder. Accordingly, a sample comprising the platelet proteome of a subject is provided and the phosphorylation level of at least one member of the cAMP/PKA-pathway and/or at least one member of the phospholipase C/PKC-pathway is compared to the phosphorylation level of the corresponding pathway member of a reference sample. Specifically, said reference sample is an earlier sample comprising the platelet proteome of the subject. A difference of at least 1.5 fold in the phosphorylation level of the at least member of the cAMP/PKA-pathway and/or the phospholipase C/PKC-pathway which indicates a decrease in platelet reactivity indicates treatment success of a vascular disease. A difference of at least 1.5 fold in the phosphorylation level of the at least one member of the cAMP/PKA-pathway and/or the phospholipase C/PKC-pathway which indicates an increase in platelet reactivity indicates treatment success of a bleeding disorder.

The term "vascular disease" as used herein refers to a number of diseases that affect the heart and circulatory system. The term vascular disease encompasses diseases and conditions including, but not limited to, coronary artery disease, coronary atherosclerosis, myocardial infarction, stroke, thrombosis, coronary occlusion, hypertensive heart disease, hypertension, peripheral artery disease and diabetes, specifically type II diabetes.

The term "thrombosis" as used herein refers to the formation or presence of coagulated blood or a thrombus attached to the site of formation or has embolized to other parts of the circulation system. A thrombus is an aggregation of blood factors, primary platelets and fibrin with entrapment of cellular elements, frequently causing vascular obstruction at the point of its formation. Conditions associated with thrombus formation include, but are not limited to, pulmonary embolism, thrombosis of the kidneys, myocardial infarction, deep venous thrombosis, venous thrombosis, arterial thrombosis, bacterial embolus, portal vein thrombosis (liver), jugular vein thrombosis, Budd-Chiari syndrome, Paget-Schroetter disease, neurovascular disorder and stroke.

The term "bleeding disorder" as used herein refers to a group of conditions that result when the blood cannot clot properly. Unlike normal clotting, platelets are not activated and cannot stick together to form a plug at the site of an injured blood vessel. Examples for bleeding disorders include but are not limited to hemophilia A, hemophilia B, factor I deficiency, factor II deficiency, factor V deficiency, factor VII deficiency, factor X deficiency, factor XI deficiency, factor XII deficiency, factor XIII deficiency and von Willebrand's disease.

The term "administration" or "administering" as used herein means providing, contacting and/or delivery of an antiplatelet agent, or clotting factor concentrate by any appropriate route to achieve the desired effect. Such appropriate route of administration may be oral, intravenous, topical, or as aerosol or as suppository.

The term "antiplatelet agent" refers to a member of a class of pharmaceuticals able to decrease platelet function and inhibit thrombus formation. Antiplatelet agents impair the ability of mature platelets to perform their normal physiological function. Examples of antiplatelet agents include but are not limited to irreversible cyclooxygenase inhibitors such as aspirin and Triflusal, adenosine diphosphate (ADP) receptor inhibitors such as clopidogrel, prasugrel, ticagrelor and ticlopidine, phosphodiesterase inhibitors such as cilostazol, protease-activated receptor-1 (PAR-1) antagonists such as vorapaxar, glycoprotein IIB/IIIA inhibitors such as abciximab, eptifibatide and tirofiban, adenosine reuptake inhibitors such as dipyridamole, thromboxane inhibitors, thromboxane synthase inhibitors and thromboxane receptor antagonists such as terutroban.

According to a specific embodiment a kit of parts is provided which comprises capturing reagents, preferably antibodies, capable of capturing one or more, or all, of phosphorylated and/or unphosphorylated LASP1, SKAP2, ZYX, RGS18, CALD, NAP1L1, PLEK and ILK, and optionally, a reference sample, and detection reagents for the detection of the phosphorylated and/or unphosphorylated proteins of a), and an instruction leaflet comprising information on the correlation of increased or decreased phosphorylation levels of at least one of LASP1, SKAP2, ZYX, RGS18, CALD1, NAP1L1, PLEK and ILK with increased or decreased platelet reactivity potential corresponding to vascular disease and bleeding disorder.

Preferably, said capturing reagents are primary binding proteins specifically recognizing phosphorylated LASP1, SKAP2, ZYX, RGS18, CALD, NAP1L1, PLEK or ILK. Specifically, said binding proteins are selected from the group consisting of antibodies, antibody fragments and peptides. Antibody fragments can be any of a Fab, Fv, scFv, dAb, F(ab)2 or Fcab fragment, or any other possible binding entity, as long as it specifically binds the phosphorylated protein.

Upon platelet inactivation, e.g. following prostacyclin (PGI₂) treatment, ZYX is phosphorylated at Ser169, Thr469, Ser505 and Ser545. Preferably, the capturing and/or detection reagent used in the *in vitro* method provided herein and in the kit provided herein is an antibody or antibody fragment, which specifically binds ZYX phosphorylated at Ser169, Thr469, Ser505 and/or Ser545.

Upon platelet activation, e.g. following ADP or TRAP-6 treatment, PLEK is phosphorylated at Thr54 and Ser53. Preferably, the capturing and/or detection reagent used in the *in vitro* method provided herein and in the kit provided herein is an antibody or antibody fragment, which specifically binds PLEK phosphorylated at Thr54 and Ser53.

Preferably, detection reagents for the detection of the phosphorylated and/or unphosphorylated proteins are binding proteins, preferably antibodies, also called secondary binding proteins, specifically recognizing either the captured proteins or the capturing reagents, e.g. primary binding proteins, and which comprise a detectable tag such as fluorogenic, chromogenic or chemiluminescent tags. Preferably, the detection reagent is a fluorescently labelled antibody or an enzymatically labelled antibody. Preferably, luciferase, alkaline phosphatase or horseradish peroxidase are used as enzymatic labels of proteins.

According to a specific embodiment of the kits provided herein, the detection reagent is an enzymatically labelled antibody and the kit further comprises enzymatic substrates such as luciferin, coelenterazine, ABTS (the diammonium salt of 2,2'-azino-di-(3-ethylbenzthiazoline-6-sulfonic acid)), PNPP (p-nitrophenyl phosphate), OPD (peroxidase ortho-phenylenediamine) or TMB (3,3',5,5'-tetramethylbenzidine).

With the kit of parts an instruction leaflet is provided in electronic or printed form, which comprises information on the correlation of the phosphorylation level of LASP1, SKAP2, ZYX, RGS18, CALD, NAP1L1, PLEK and/or ILK with diagnosis or treatment success of vascular disease or bleeding disorder.

Specifically, the instruction leaflet comprises the information that an increase of at least 1.5 fold in the phosphorylation level of CALD1, NAP1L1 and PLEK compared to the phosphorylation level of CALD1, NAP1L1 and PLEK of a healthy subject or a pool of healthy subjects or a pre-determined phosphorylation level indicated in the leaflet is indicative of increased platelet reactivity potential, wherein increased platelet reactivity potential identifies the subject as being at risk of or having a vascular disease.

Specifically, the instruction leaflet further comprises the information that a decrease of at least 1.5 fold in the phosphorylation level of LASP1, SKAP2, ZYX, RGS18 and ILK compared to the phosphorylation level of LASP1, SKAP2, ZYX, RGS18 and ILK of a healthy subject or a pool of healthy subjects or a pre-determined phosphorylation level indicated in the leaflet is indicative of increased platelet reactivity potential, wherein increased platelet reactivity potential identifies the subject as being at risk of or having a vascular disease.

Specifically, the instruction leaflet comprises the information that a decrease of at least 1.5 fold in the phosphorylation level of CALD1, NAP1L1 and PLEK compared to the phosphorylation level of CALD1, NAP1L1 and PLEK of a healthy subject or a pool of healthy subjects or a pre-determined phosphorylation level indicated in the leaflet is indicative of decreased platelet reactivity potential, wherein decreased platelet reactivity potential identifies the subject as being at risk of or having a bleeding disorder.

Specifically, the instruction leaflet further comprises the information that an increase of at least 1.5 fold in the phosphorylation level of LASP1, SKAP2, ZYX, RGS18 and ILK compared to the phosphorylation level of LASP1, SKAP2, ZYX, RGS18 and ILK of a healthy subject or a pool of healthy subjects or a pre-determined phosphorylation level indicated in the leaflet is indicative of decreased platelet reactivity potential, wherein decreased platelet reactivity potential identifies the subject as being at risk of or having a bleeding disorder.

Specifically, the instruction leaflet further comprises the information that a decrease of at least 1.5 fold in the phosphorylation level of CALD1, NAP1L1 and PLEK compared to the phosphorylation level of CALD1, NAP1L1 and PLEK of an earlier sample of the test subject or a pre-determined phosphorylation level indicated in the leaflet is indicative of decreased platelet reactivity potential, wherein decreased platelet reactivity potential indicates that the treatment for a vascular disease is successful.

Specifically, the instruction leaflet further comprises the information that an increase of at least 1.5 fold in the phosphorylation level of LASP1, SKAP2, ZYX, RGS18 and ILK compared to the phosphorylation level of LASP1, SKAP2, ZYX, RGS18 and ILK of an earlier sample of the test subject or a pre-determined phosphorylation level indicated in the leaflet is indicative of decreased platelet reactivity potential, wherein decreased platelet reactivity potential indicates that the treatment for a vascular disease is successful.

Specifically, the instruction leaflet further comprises the information that an increase of at least 1.5 fold in the phosphorylation level of CALD1, NAP1L1 and PLEK compared to the phosphorylation level of CALD1, NAP1L1 and PLEK of an earlier sample of the test subject or a pre-determined phosphorylation level indicated in the leaflet is indicative of increased platelet reactivity potential, wherein increased platelet reactivity potential indicates that the treatment for a bleeding disorder is successful.

Specifically, the instruction leaflet further comprises the information that a decrease of at least 1.5 fold in the phosphorylation level of LASP1, SKAP2, ZYX, RGS18 and ILK compared to the phosphorylation level of LASP1, SKAP2, ZYX, RGS18 and ILK of an earlier sample of the test subject or a pre-determined phosphorylation level indicated in the leaflet is indicative of increased platelet reactivity potential, wherein increased platelet reactivity potential indicates that the treatment for a bleeding disorder is successful.

A treatment is successful, when the platelet reactivity potential progresses towards physiological levels, specifically, when the phosphorylation level of LASP1, SKAP2, ZYX, RGS18, CALD1, NAP1L1, PLEK and/or ILK progressed towards physiological levels compared to a reference sample. Treatment success does not necessarily mean that phosphorylation levels in a sample comprising the platelet proteome of a test subject are the same as the phosphorylation levels in a sample comprising the platelet proteome of a healthy subject or a pool of healthy subjects.
The present invention further comprises the following items:
1. An *in vitro* method of identifying abnormal platelet reactivity potential of a subject, comprising the steps of:
   i) providing a sample comprising the platelet proteome of said subject, and
   ii) measuring the phosphorylation level of at least one member of the cAMP/ protein kinase A (PKA)-pathway and comparing the phosphorylation level of said at least one cAMP/PKA-pathway member with the phosphorylation level of the corresponding at least one cAMP/PKA-pathway member in a reference sample, and/or
   iii) measuring the phosphorylation level of at least one member of the phospholipase C/protein kinase C (PKC)-pathway and comparing the phosphorylation level of said at least one phospholipase C/PKC-pathway member with the phosphorylation level of the corresponding at least one phospholipase C/PKC-pathway member in a reference sample,
   wherein a difference in the phosphorylation level of at least 1.5 fold in ii) and/or iii) is indicative of abnormal platelet reactivity potential.
2. The *in vitro* method of item 1, wherein the abnormal platelet reactivity is correlated with vascular disease or bleeding disorder.
3. The *in vitro* method of item 1 or 2, wherein the at least one member of the cAMP/PKA-pathway is selected from the group consisting of LIM and SH3 domain protein 1 (LASP1), Src kinase-associated phosphoprotein 2 (SKAP2), Zyxin (ZYX), regulator of G-protein signaling 18 (RGS18), Caldesmon 1 (CALD1) and Nucleosome assembly protein 1-like 1 (NAP1L1).
4. The *in vitro* method of any of items 1 to 3, wherein a decrease of at least 1.5 fold in the phosphorylation level of any one or more of LASP1, SKAP2, ZYX and RGS18 indicates increased platelet reactivity potential.
5. The *in vitro* method of any of items 1 to 4, wherein an increase of at least 1.5 fold in the phosphorylation level of CALD1 and/or NAP1L1 indicates increased platelet reactivity potential.
6. The *in vitro* method of item 1 or 2, wherein the at least one member of the phospholipase C/PKC-pathway is selected from the group consisting of pleckstrin (PLEK) and integrin-linked protein kinase (ILK).
7. The *in vitro* method of item 6, wherein an increase of at least 1.5 fold in the phosphorylation level of PLEK indicates increased platelet reactivity potential.
8. The *in vitro* method of item 6, wherein a decrease of at least 1.5 fold in the phosphorylation level of ILK indicates increased platelet reactivity.
9. The in vitro method of item 1 or 2, wherein the reference sample is an earlier sample of the same subject or a sample of a healthy subject or of a pool of healthy subjects.
10. The *in vitro* method of item 1 or 2 for the diagnosis of a vascular disorder of a subject, comprising the steps of:
   a) providing a sample comprising the platelet proteome of said subject,
   b) measuring the phosphorylation level of at least one member of the cAMP/PKA-pathway selected from the group consisting of LASP1, SKAP2, ZYX, RGS18, CALD1 and NAP1L1,
   c) comparing the phosphorylation level of said at least one cAMP/PKA-pathway member with the phosphorylation level of the corresponding at least one cAMP/PKA-pathway member in a reference sample of a healthy subject or a pool of healthy subjects,
      i) wherein a decrease of at least 1.5 fold in the phosphorylation level of any one or more of LASP1, SKAP2, ZYX and RGS18 indicates increased platelet reactivity potential,
      ii) wherein an increase of at least 1.5 fold in the phosphorylation level of CALD1 and/or NAP1L1 indicates increased platelet reactivity potential, and
   wherein increased platelet reactivity potential identifies the subject as being at risk of or having a vascular disorder.
11. The *in vitro* method of item 1 or 2 for the identification of treatment success of a subject having a vascular disorder, comprising the steps of:
   a) providing a sample comprising the platelet proteome of said subject,
   b) measuring the phosphorylation level of at least one member of the cAMP/PKA-pathway selected from the group consisting of LASP1, SKAP2, ZYX, RGS18, CALD1 and NAP1L1,
   c) comparing the phosphorylation level of said at least one cAMP/PKA-pathway member with the phosphorylation level of the corresponding at least one cAMP/PKA-pathway member in a reference sample, which is an earlier sample of said subject,
      i) wherein an increase of at least 1.5 fold in the phosphorylation level of any one or more of LASP1, SKAP2, ZYX and RGS18 indicates decreased platelet reactivity potential,
      iii) wherein a decrease of at least 1.5 fold in the phosphorylation level of CALD1 and/or NAP1L1 indicates decreased platelet reactivity potential, and
   wherein decreased platelet reactivity potential is indicative of treatment success.
12. The *in vitro* method of item 1 or 2 for the diagnosis of a vascular disorder of a subject, comprising the steps of:
   a) providing a sample comprising the platelet proteome of said subject,
   b) measuring the phosphorylation level of at least one member of the phospholipase C/PKC-pathway selected from the group consisting of PLEK and ILK,
   c) comparing the phosphorylation level of said at least one phospholipase C/PKC-pathway member with the phosphorylation level of the corresponding at least one phospholipase C/PKC-pathway member in a reference sample of a healthy subject or a pool of healthy subjects,
      i) wherein an increase of at least 1.5 fold in the phosphorylation level of PLEK indicates increased platelet reactivity potential,
      iii) wherein a decrease of at least 1.5 fold in the phosphorylation level of ILK indicates increased platelet reactivity potential, and
   wherein increased platelet reactivity potential identifies the subject as being at risk of or having a vascular disorder.
13. The *in vitro* method of item 1 or 2 for the identification of treatment success of a subject having a vascular disorder, comprising the steps of:
   a) providing a sample comprising the platelet proteome of said subject,
   b) measuring the phosphorylation level of at least one member of the phospholipase C/PKC-pathway selected from the group consisting of PLEK and ILK,
   c) comparing the phosphorylation level of said at least one phospholipase C/PKC-pathway member with the phosphorylation level of the corresponding at least one phospholipase C/PKC-pathway member in a reference sample, which is an earlier sample of said subject,
      i) wherein a decrease of at least 1.5 fold in the phosphorylation level of PLEK indicates decreased platelet reactivity potential,
      iii) wherein an increase of at least 1.5 fold in the phosphorylation level of ILK indicates decreased platelet reactivity potential, and
   wherein decreased platelet reactivity potential is indicative of treatment success.
14. The *in vitro* method of item 1 or 2 for the diagnosis of a bleeding disorder of a subject, comprising the steps of:
   a) providing a sample comprising the platelet proteome of said subject,
   b) measuring the phosphorylation level of at least one member of the cAMP/PKA-pathway selected from the group consisting of LASP1, SKAP2, ZYX, RGS18, CALD1 and NAP1L1,
   c) comparing the phosphorylation level of said at least one cAMP/PKA-pathway member with the phosphorylation level of the corresponding at least one cAMP/PKA-pathway member in a reference sample of a healthy subject or a pool of healthy subjects,
      i) wherein an increase of at least 1.5 fold in the phosphorylation level of any one or more of LASP1, SKAP2, ZYX and RGS18 indicates decreased platelet reactivity potential,
      iii) wherein a decrease of at least 1.5 fold in the phosphorylation level of CALD1 and/or NAP1L1 indicates decreased platelet reactivity potential, and
   wherein decreased platelet reactivity potential identifies the subject as being at risk of or having a bleeding disorder.
15. The *in vitro* method of item 1 or 2 for the identification of treatment success of a subject having a bleeding disorder, comprising the steps of:
   a) providing a sample comprising the platelet proteome of said subject,
   b) measuring the phosphorylation level of at least one member of the cAMP/PKA-pathway selected from the group consisting of LASP1, SKAP2, ZYX, RGS18, CALD1 and NAP1L1,
   c) comparing the phosphorylation level of said at least one cAMP/PKA-pathway member with the phosphorylation level of the corresponding at least one cAMP/PKA-pathway member in a reference sample, which is an earlier sample of said subject,
      i) wherein a decrease of at least 1.5 fold in the phosphorylation level of any one or more of LASP1, SKAP2, ZYX and RGS18 indicates increased platelet reactivity potential,
      iii) wherein an increase of at least 1.5 fold in the phosphorylation level of CALD1 and/or NAP1L1 indicates increased platelet reactivity potential, and
   wherein increased platelet reactivity potential is indicative of treatment success.
16. The *in vitro* method of item 1 or 2 for the diagnosis of a bleeding disorder of a subject, comprising the steps of:
   a) providing a sample comprising the platelet proteome of said subject,
   b) measuring the phosphorylation level of at least one member of the phospholipase C/PKC-pathway selected from the group consisting of PLEK and ILK,
   c) comparing the phosphorylation level of said at least one phospholipase C/PKC-pathway member with the phosphorylation level of the corresponding at least one phospholipase C/PKC-pathway member in a reference sample of a healthy subject or a pool of healthy subjects,
      i) wherein a decrease of at least 1.5 fold in the phosphorylation level of PLEK indicates decreased platelet reactivity potential,
      iii) wherein an increase of at least 1.5 fold in the phosphorylation level of ILK indicates decreased platelet reactivity potential, and
   wherein decreased platelet reactivity potential identifies the subject as being at risk of or having a bleeding disorder.
17. The *in vitro* method of item 1 or 2 for the identification of treatment success of a subject having a bleeding disorder, comprising the steps of:
   a) providing a sample comprising the platelet proteome of said subject,
   b) measuring the phosphorylation level of at least one member of the phospholipase C/PKC-pathway selected from the group consisting of PLEK and ILK,
   c) comparing the phosphorylation level of said at least one phospholipase C/PKC-pathway member with the phosphorylation level of the corresponding at least one phospholipase C/PKC-pathway member in a reference sample, which is an earlier sample of said subject,
      i) wherein an increase of at least 1.5 fold in the phosphorylation level of PLEK indicates increased platelet reactivity potential,
      iii) wherein a decrease of at least 1.5 fold in the phosphorylation level of ILK indicates increased platelet reactivity potential, and
   wherein increased platelet reactivity potential is indicative of treatment success.
18. The *in vitro* method of any of items 9 to 12, wherein the bleeding disorder is selected from the group consisting of hemophilia A, hemophilia B, factor I deficiency, factor II deficiency, factor V deficiency, factor VII deficiency, factor X deficiency, factor XI deficiency, factor XII deficiency, factor XIII deficiency and von Willebrand's disease.
19. The *in vitro* method of any of items 13 to 16, wherein the vascular disease is selected from the group consisting of coronary artery disease, coronary atherosclerosis, myocardial infarction, stroke, thrombosis, coronary occlusion, hypertensive heart disease, hypertension, peripheral artery disease and diabetes, specifically type II diabetes.
20. The *in vitro* method of any of items 9 to 18, wherein the subject identified as being at risk of having or having a bleeding disorder is subjected to substitution treatment or administered clotting factor concentrate and wherein the subject identified as being at risk of having or having a vascular disease is administered an antiplatelet agent.
21. The *in vitro* method of item 19, wherein the antiplatelet agent is selected from the group consisting of irreversible cyclooxygenase inhibitors such as aspirin and Triflusal, adenosine diphosphate (ADP) receptor inhibitors such as clopidogrel, prasugrel, ticagrelor and ticlopidine, phosphodiesterase inhibitors such as cilostazol, protease-activated receptor-1 (PAR-1) antagonists such as vorapaxar, glycoprotein IIB/IIIA inhibitors such as abciximab, eptifibatide and tirofiban, adenosine reuptake inhibitors such as dipyridamole, thromboxane inhibitors, thromboxane synthase inhibitors and thromboxane receptor antagonists such as terutroban.
22. The *in vitro* method of any of items 1 to 16, wherein the sample is selected from the group consisting of whole blood, plasma, platelet rich plasma (PRP) and platelet enriched fraction,
23. The *in vitro* method of any of items 1 to 16, wherein the platelet proteome is derived from a sample by gel-filtration and subsequent precipitation under conditions avoiding further platelet activation.
24. A kit of parts comprising
   a) capturing reagents, preferably antibodies, capable of capturing at least one of phosphorylated and/or unphosphorylated LASP1, SKAP2, ZYX, RGS18, CALD, NAP1L1, PLEK and ILK, and
   b) optionally, a reference sample, and
   c) detection reagents for the detection of the phosphorylated and/or unphosphorylated proteins of a), and
   d) an instruction leaflet comprising information on the correlation of increased or decreased phosphorylation levels of at least one of LASP1, SKAP2, ZYX, RGS18, CALD1, NAP1L1, PLEK and ILK with increased or decreased platelet reactivity potential corresponding to vascular disease and bleeding disorder.
25. A kit of parts of a panel of platelet proteins comprising
   a) capturing reagents, preferably antibodies, capable of capturing two or more of, preferably all of, phosphorylated and/or unphosphorylated LASP1, SKAP2, ZYX, RGS18, CALD, NAP1L1, PLEK and ILK, and
   b) optionally, a reference sample, and
   c) detection reagents for the detection of the phosphorylated and/or unphosphorylated proteins of a), and
   d) an instruction leaflet comprising information on the correlation of increased or decreased phosphorylation levels of two or more of, preferably all of LASP1, SKAP2, ZYX, RGS18, CALD, NAP1L1, PLEK and ILK with increased or decreased platelet reactivity potential corresponding to vascular disease and bleeding disorder.

The present invention is further illustrated by the following examples without being limited thereto. The Examples which follow are set forth to aid in the understanding of the invention but are not intended to, and should not be construed to limit the scope of the invention in any way. The Examples do not include detailed descriptions of conventional methods. Such methods are well known to those of ordinary skill in the art.

### EXAMPLES

To enlarge the biomarker repertoire used to study disruptions of platelet reactivity, the non-secretory platelet proteome after *in vitro*-activation and -inactivation was compared with their respective controls using unbiased fluorescence two-dimensional differential in-gel electrophoresis (2D-DIGE).

Importantly, the concentrations of platelet activators and inactivators needed to induce these two main regulatory conditions were selected from typical protocols of functional platelet studies.¹⁹⁻²² Notably, instead of the usual centrifugation step, proteins of *in vitro* treated gel-filtered platelet suspension were extracted by TCA-precipitation²³ to "freeze" them in their particular status. According to the methods applied, only non-secretory platelet proteome changes were quantified since the granule release was not separated. Two-dimensional in-gel electrophoresis enables a simultaneous investigation of proteins and their different protein species, which mainly arise from post-translational modifications. The terminology "protein species" is used to describe proteins which arise from the same amino acid sequence but have been modified post-translationally.²⁴ Using 2D-DIGE, several protein spots were identified to be specific to either activated or inactivated platelets. By measuring their abundance, some of these proteins were found to be more powerful than established biomarkers in predicting the platelet activation and inactivation potential.

The results indicated that non-secretory proteome imprints were qualitatively and quantitatively more pronounced upon activation than during activation. These alterations were, to a large extent, caused by changes in the phosphorylation profiles in 17 out of 31 proteins. Remarkably, while VASP reached a 1.3-fold increase in phosphorylation levels in inactivated platelets, several of the aforementioned proteins showed significantly higher inactivation-induced phosphorylation levels, including LIM and SH3 domain protein 1 (6.7-fold) and Src kinase-associated phosphoprotein 2 (4.6-fold). Moreover, phosphorylation levels of pleckstrin (PLEK) and of integrin-linked protein kinase (ILK) species were strongly associated with P-selectin surface expression. The discrimination power between activation and inactivation was more pronounced for phosphorylated PLEK (3.77 Cohen's d effect size) and for dephosphorylated ILK (3.79) species than for P-selectin (2.35). Therefore, these data revealed new and powerful biomarkers to measure platelet reactivity potential.

### Example 1: Identification of Markers of Platelet Reactivity Potential

### a) Methods

### Chemicals

Adenosine diphosphate (ADP), prostacyclin (PGI2), Thrombin receptor-activating peptide-6 (TRAP-6), lodoacetamide (IAA) were all purchased from Sigma Aldrich, St. Louis, MO, USA; CTAD blood tubes (3.5 ml, 0.129 mM trisodium citrate, 15 mM theophylline, 3.7 mM adenosine, 0.198 mM dipyridamole) and 0.129 mM Sodium-citrate blood tubes were obtained from Greiner Bio-One, Kremsmunster, Austria; Bradford Coomassie Plus kit (Pierce Biotechnology, USA); Dithiothreitol (DTT) (Merck, Germany); Econo-Pac Chromatography Columns 15 mm (Bio Rad, USA); Sepharose-2B (GE Healthcare, Uppsala, Sweden); calcium-free Dubecco's PBS-buffer (GIBCO, Paisley, Scotland, UK); TCA (Trichloracetic acid 6.1N solution, Sigma Aldrich, St. Louis, MO, USA).

### Antibodies and Enzymes

Lambda phosphatase and reaction buffer Lot 0110803 (New England BioLabs, MA, USA); Trypsin (Promega, WI, USA); Donkey Anti-Goat IgG (H+L) LOT 134531 (Jackson ImmunoResearch Laboratories Inc., PA, USA); VASP fix ("Platelet Solutions", Nottingham, UK); CD62P (anti-human CD62P, clone AK4, BioLegend, CA, USA).

### Blood Sampling and Platelet Isolation

The study included twelve healthy volunteers aged 31.7±7.1 years (mean ± SD), who had not taken any antiplatelet medication for at least 14 days prior to blood sampling. The study was approved by the Ethics Committtee of the Medical University of Vienna and was conducted in accordance with the Declaration of Helsinki.

Blood was drawn from the antecubital vein (21 G needle) without stasis into sodium-citrate tubes (3.5 ml containing 0.129 mM citrate) as well as CTAD tubes (3.5 ml, containing 0.129 mM trisodium-citrate, 15 mM theophylline, 3.7 mM adenosine, 0.198 mM dipyridamole). Blood tubes were centrifuged at 120xg for 20 minutes at room temperature to obtain platelet-rich plasma (PRP). A graphical experimental design for *in vitro* treatments is shown in Figure 1.

For inactivation, citrated PRP was incubated with PGI₂ (0.4 µM final) for 5 minutes prior to gel-filtration, while PRP from CTAD blood tubes was left untreated. A PGI₂ concentration of 0.1-0.5 µM is normally used to generate washed platelets.¹⁻³ Commercially available CTAD blood tubes increase cyclic adenosine monophosphate (cAMP) levels to prevent platelet activation during blood sampling and subsequent processing steps.⁴ To gently purify platelets from plasma proteins by size-exclusion chromatography,⁵ 1 ml PRP was applied onto a 12 cm sepharose-2B packed column pre-equilibrated and eluted in PBS buffer. Gel-filtered platelets (GFP) were caught at the end of the column and precipitated by mixing 3:1 (v/v) with 6.1N TCA solution (containing 80 mM DTT). Alternatively, PGI₂ was added not to the platelet rich plasma but to the gel-filtered platelets suspension, which induced comparable changes in the proteome as seen in PRP+PGI₂ and thus showed that the observed protein abundance changes were not simply derived from protein handling during gel-filtration.

For activation, 15 µM TRAP-6 and 5 µM ADP was chosen to evoke a strong and moderate activation response, respectively. GFP from citrated PRP, was incubated with ADP (5 µM) or TRAP-6 (15 µM) for 15 minutes. Gel-filtration constitutes a gentle method to isolate plateletS^{6,7}, however, activated platelets cannot pass the sepharose column and get stuck, resulting in a diminished platelet yield. Therefore, it was decided to activate platelets after gel-filtration.

Stimulations were stopped by TCA/DTT precipitation after 15 minutes. For baseline values, corresponding citrated PRP was gel-filtered and immediately precipitated. After one hour at 4°C, the TCA-extracted platelet proteins from all conditions were pelleted at 20,000 xg for 10 minutes at 4°C and washed four times (20,000 xg, 10 minutes, 4°C) with acetone.

### Platelet Proteome Analysis by Fluorescence two-dimensional Gel Electrophoresis (2D-DIGE)

Resolubilization of precipitated proteins, determination of protein concentration, preparation of the internal standard and labeling with fluorescent cyanine dyes (Cy2, Cy3, Cy5) were performed as described previously.²⁵ Briefly, the TCA-precipitated and acetone-washed platelet proteins were resolubilized in urea-sample buffer (7 M urea, 2 M Thiourea, 4% CHAPS, 20 mM Tris-HCl pH 8.5) and fluorescence-labeled prior to 2D-separation. For the normalization process, an internal standard (IS) of platelet proteins from all treatment conditions was prepared. This IS, labeled in Cy2 and added to every 2D-gel, enables a highly accurate qualitative and quantitative comparison of all samples and treatments.^{26,27} The 2D-DIGE analysis was performed in the pH-ranges 4-7 and 6-9, respectively.

For the acidic side, 36 µg protein (2 x 12 µg sample + 1 x 12 µg internal standard) were passively rehydrated on 24 cm pH 4-7 IPG-Dry-Strips in a solution containing 7 M urea, 2 M thiourea, 4 % CHAPS, 70 mM DTT and 0.5 % ampholyte pH 4-7.

For the alkaline side, 24 cm pH 6-9 IPG-Dry-Strips (GE Healthcare, Uppsala, Sweden) were soaked in a rehydration solution containing 7 M urea, 2 M thiourea, 4 % CHAPS, 150 mM DTT and 2 % ampholytes pH 6-10 prior to isoelectric focusing. Afterward, 3 x 12 µg of labeled protein samples were applied by "cup-loading" via the acidic side and the isoelectric focusing was carried out until 30 kVh was reached.

The following SDS-PAGE was performed with an 11.5 % acrylamide gel at 35 V for 1 hour, 50 V for 1.5 hours and 110 V for 16.5 hours at 10°C in an Ettan DALTsix electrophoresis chamber (GE Healthcare, Uppsala, Sweden). The gels were scanned afterward with a resolution of 100 µm using a Typhoon 9410 imager (GE Healthcare, Uppsala, Sweden).

### Analysis of Platelet Activation by Flow Cytometry

To monitor platelet activation status, the commonly used platelet surface activation marker CD62P was determined. Therefore, untreated and treated (PGI2, CTAD, ADP, TRAP-6) gel-filtered platelets were incubated with a phycoerythrin (PE)-labeled antibody against CD62P (dilution 1:40) for 20 minutes. Afterward, platelets were fixed with paraformaldehyde (1 % final concentration) and analyzed by flow cytometry using a BD Accuri flow cytometer and BD AccuriC6 analysis software (Becton Dickinson, NJ, USA). Read-outs are given as fold changes (treated vs. untreated). Basal CD62P expression of citrated GFP was considered as baseline (100 %). To detect differences between citrate and inactivation (PGI2, CTAD) or activation (ADP, TRAP-6), respectively, a non-parametric one sample Wilcoxen-rank sum test was performed. Significance level (two-sided) was set at p < 0.05 for all tests. SPSS Statistics 20.0 (IBM, Chicago, USA) was used.

### VASP Phosphorylation flow cytometry assay to validate 2D-DIGE results

To validate the phospho-profiles of VASP upon PGI₂ and CTAD treatment revealed by 2D-DIGE, the phosphorylation site Ser157 of VASP was assessed by flow cytometry. Blood samples from three healthy volunteers were freshly drawn into citrated and CTAD blood tubes and processed to generate gel-filtered platelets (as described above). Gel-filtered platelets from citrated, PGI₂ (0.4µM) and CTAD treated platelets were subjected to the ready-to-use *VASPfix-* assay ("Platelet Solutions", Nottingham, UK). 4 µl of gel-filtered platelets was forcefully mixed with 25 µl *VASPfix-*solution to perforate the platelet membrane and make VASP accessible. After a one hour incubation in the dark, flow cytometry was performed (Accuri C6, Becton Dickinson). VASP-beads were detected by their allophycocyanin (APC-) fluorescence. VASP antibody against Ser157 was detected by its fluorescein isothiocyanate (FITC-) fluorescence on APC-gated beads. Values are represented as median fluorescence intensity (Figure 2). SPSS Statistics 20.0 (IBM, Chicago, USA) was used.

### 2D-DIGE Image Analysis

For spot detection and inter-gel matching the "DeCyderTM 6.5.14" software (GE Healthcare, Uppsala, Sweden) was used. Spot intensities were normalized to remove differences in staining intensities and to eliminate running differences between 2D gel images. The obtained values (Standardized Abundance - SA) were subsequently used for the statistical analysis and are referred to as fold change (FC).

### Identification of Protein Spots by Mass Spectrometry

For MS-based identifications, 250 µg unlabeled proteins were separated on a preparative two-dimensional gel. Proteins were visualized by MS-compatible silver staining.⁸ Spots differentially regulated on the basis of 2D-DIGE analysis were excised with a scalpel, destained, reduced, alkylated and tryptically digested. Peptides were applied onto a Dionex Ultimate 3000 RSLC nano-HPLC system (Thermo Scientific) equipped with a pre-concentration and desalting cartridge where 0.1 % TFA was used as transport liquid. Peptides were separated on a Acclaim PepMap RSLC column (250 mm × 75 µm, C18, 2 µm, 100Å; Thermo Scientific) through elution with a 59 minute linear gradient from 4 to 45 % of solvent B using flow rate of 500 nL/minute (solvent A: 0.1 % TFA, solvent B: acetonitrile/ddH₂O/formic acid, 80/20/0.1 % (v/v/v)) and directly subjected to the mass spectrometer. In-line bottom-up proteomics was performed on a QqTOF mass spectrometer oTOF compact from Bruker Daltonics (Billerica, MA, United States) equipped with nano-flow CaptiveSpray ionization device and using the oTOF control software v3.4 (build16). High mass accuracy was achieved by using an internal calibrant (hexakis-(1H,-1H,-4H-hexafluorobutyloxy)-phosphazine (Agilent Technologies)) during the runs. The cycle time for MS1 and MS/MS fragmentation was 3 seconds, scan range for precursor recording was set to 50-2,200 m/z, spectra rate was set to 2 Hz, dry gas was set to 3 L/minute and the dry temperature was set to 150°C. The peptide assignment to proteins was conducted with the Proteinscape software v.3.1.5 474 (Build 0140711-1459, Bruker Daltonics) which used the Mascot algorithm version 2.5 (Matrixscience, MA) and peak lists generated with the Compass Data Analysis software v4.2 (Build 395, Bruker Daltonics). Parameters for the protein search were: enzyme specificity: trypsin; species: human; database: UniProtKB/Swiss-Prot; peptide tolerance ± 10 ppm; MS/MS tolerance ± 0.05 Da; number of considered 13C atoms 1; charge states 1+-3+; up to two missed cleavage; fixed modifications carbamidomethylation (C), variable modifications: deamidation (N, Q), oxidation (M), phosphorylation (S, T, Y), acetylation (K, N-term). The search algorithm probability score was set at p < 0.05.

### Characterization of the Platelet Phospho-Proteome by A-Phosphatase Treatment

To determine phosphorylated protein species, we employed A-phosphatase treatment as described previously.²⁸ Briefly, precipitated GFP aliquots were resolubilized in lysis buffer (7 M Urea, 2 M Thiourea, 4% Chaps, 1% DTT), followed by a sonication step (10 minutes, 4°C), overnight incubation (800 rpm, 4°C), and determination of the protein concentration using a Bradford kit. Subsequently, two aliquots a 200 µg of each sample condition (PGI₂/CTAD) were mixed with 5 µl of 10% SDS. Thereafter, samples were filled up to 500 µl with a reaction mix containing 5 mM DTT, 2 mM MnCl₂, and 1xλ-phosphatase buffer. One PGI₂ and CTAD aliquot were incubated overnight with 100 units of A-PPase (30°C, 300rpm). Thereafter, all samples (±λ PPase) were precipitated with TCA/DTT.

### Statistics

Fold changes (FC) of standardized protein values (treatment/IS) were calculated between intervention groups (PGI₂, CTAD, ADP, TRAP-6) and the untreated control group (citrate). Protein spots were considered relevant if 1) spots were matched in more than 90% of all 2D images (n = 24) and 2) if FC were > 20% as detected between baseline (citrate) and at least one of the intervention groups PGI₂, CTAD, ADP, TRAP-6. After this filtering, 495 of 5,906 protein spots in the pH range 4-9 were considered relevant. Log-transformed FC (495 spots) were evaluated by a repeated ANOVA conducted as a linear mixed model with "group" (citrate, PGI₂, CTAD, ADP, TRAP-6) as repeated factor. The multiplicity was controlled by the Benjamini-Hochberg procedure (FDR < 0.05). SPSS Statistics 20.0 (IBM, Chicago, USA) was used for the statistical analysis. Unless stated otherwise, all fold changes represent means.

### Biological Pathway Analysis

To gain a deeper insight into the biological function of differentially regulated proteins identified by 2DDIGE, we initially applied Reactome pathway enrichment analysis (www.reactome.org) and found "Hemostasis" followed by "Platelet activation signaling and aggregation" as pathways with the highest number of overlaps and lowest p-value/FDR corrected p-value enriched (7.5E-11/3.3E-08, 10 proteins overlap and 7.02E-11/3.31E-08, 8 proteins overlap, respectively). Next, an integrated biological database software-suite was employed (Ingenuity Pathway Analysis, IPA) in addition to extensive literature research. For the initial IPA-core analysis, proteins differentially regulated upon platelet inactivation (PGI2 and CTAD, n = 26) compared to baseline (citrate) were entered along with their average fold change and the FDR-corrected p-value to enable weighting. The corresponding highest network score (45 with 17 focus molecules) included "Cell-to-Cell Signaling and Interaction", "Hematological System Development", and "Function and Inflammatory Response" within the term "Top Disease and Functions", where PKA was a key node and PKC was inferred manually based on literature.¹⁶ Subsequently, datasets containing proteins and corresponding fold changes identified after A-phosphatase treatment (PGI₂+λ-PPase, CTAD+λ-PPase) and corresponding controls (PGI₂, CTAD) were subjected to phosphorylation analysis to predict kinases and phosphatases within the upstream-regulators-module. By applying the Pathway Building tool, proteins that were differentially regulated by platelet activation (ADP and TRAP-6, n = 5), as well as the predicted kinases and phosphatases, were added and the initial network was narrowed to the platelet system. To give an additional layer of information, the Molecule Activity Predictor tool, which uses the protein level alterations within subgroups (PGI₂, CTAD, ADP, TRAP-6) was applied to suggest functional effects on neighboring molecules. For visualization, differentially regulated proteins identified upon inactivation (PGI₂ vs. citrate and CTAD vs. citrate), or activation (ADP vs. citrate and TRAP-6 vs. citrate), were alphabetically arranged in a circular layout and connected to the central nodes PKA and PKC, respectively.

### b) Results

### Quality Control of Platelet In Vitro Models and 2D-DIGE Analysis

To assure that our platelet proteome analysis was assessed from well-defined in vitro activated and inactivated platelets, the activation marker CD62P was measured by flow cytometry. A strong platelet activation (> 90% CD62P-positive cells) was induced by TRAP-6 (15 µM) and a moderate response with ADP (5 µM, on average 50% CD62P-positive platelets). Accordingly, we found 7.36-fold and 2.53-fold more CD62P-positive cells, as compared to untreated controls, for TRAP-6 (p = 0.009) and ADP (p = 0.03), respectively (Figure 3). These increases imply that platelet preparation and activation worked well in our experimental setting. For the assessment of platelet inactivation, CD62P surface expression was evaluated after CTAD and PGI₂ (0.4 µM) treatments. Inactivated GFPs showed a reduced CD62P expression of 0.67-fold after CTAD- (p = 0.005) and 0.83-fold after PGI₂ treatment (p = 0.016) compared to untreated GFP. We also monitored the quality of our 2D-DIGE analysis regarding the biological and technical coefficient of variation (CVₜₒₜ = CV_{bio} + CV_{tech}).²⁷ In the present study, we determined a median CVₜₒₜ of 12.3% in the untreated citrated platelet proteome (5906 spots) from all included volunteers, plus a single CVₜₒₜ of 5.7% for 14-3-3γ, a low biological variation protein, confirming high-technical quality when compared to our previous platelet proteomic studies.²⁵

### Inactivation shows stronger changes than activation in the 2D-DIGE Platelet Proteome

The proteomes of activated and inactivated platelets were compared with untreated controls to gain a deeper insight into phenotypic changes reflecting their reactivity status. PGI₂ and CTAD were applied to inactivate platelets, whereas TRAP-6 and ADP were added for activation. Platelet protein extraction was performed by TCA-precipitation of the GFP-suspension to prevent any additional activation during platelet pelleting by centrifugation. This precipitation step, however, prohibited the separation of the platelet secretome. Therefore, our experiment focused on events leading to changes within the non-secretory platelet proteome. Electrophoretic analysis of precipitated proteins was performed at the pH ranges of 4-7 and 6-9. Protein abundances were evaluated by DeCyder-image analysis software and quantified as fold changes compared to untreated controls. These analyses revealed 73 differentially regulated protein spots according to our selection criteria. Upon PGI₂ addition, 65 protein spots were changed, 45 were changed by adding CTAD, with a broad overlap (44 spots) (Figure 4A). In contrast, six protein spots were regulated differently following TRAP-6 treatment compared to seven ADP-related protein spots (Figure 4B). In summary, ten times more protein spots were affected by inactivation with a median abundance difference of 11% (+1.45- vs. +1.34-median FC, p = 0.03 Figure 4A&B). Hence, inactivation footprints were qualitatively and quantitatively more prominent than activation ones, a finding that encouraged us to investigate the molecular basis for this difference in more detail.

### Platelet Proteome Changes after Inactivation/Activation are Mostly Caused by Phosphorylation

To investigate the stronger proteome changes seen in inactivated platelets compared to activated ones, the differentially regulated protein spots were identified by mass spectrometry. The results showed that several identified proteins appeared in more than one spot, giving a total of 31 distinct proteins (selected proteins shown in Table 1, Figure 4 A2&B2). These protein species, originating from a single protein, had the same molecular weight, however, migrated across the pI range, thereby displaying a chain-like pattern. 2D-DIGE images showed significantly altered platelet protein spots upon inactivation and activation. A total of 36 µg (12 µg sample Cy3-, 12 µg sample Cy5-, and 12 µg internal standard Cy2-labeled) platelet protein extracts were separated according to the isoelectric point (pI) and the molecular weight (MW) in the pH ranges 4-7 and 6-9. Altered proteins were identified by mass spectrometry and are outlined for inactivation and for activation with their respective UniProt "Gene" name (Table 1). Protein spots of interest were selected according to (a) protein spots matched > 90% of all 2D-DIGE gels, (b) protein abundance changes > 20% between treatment group and citrated control and (c) FDR-corrected ANOVA p-value < 0.05. For example, five different protein species with the same molecular weight but different pIs ranging from 7.19 to 9.05 were identified as VASP (Figure 5A). A similar pattern was observed for LASP1, ZYX, and SKAP2 (Figure 5B-D). Subsequently, biological database analysis (Reactome) was applied to identify the most probable pathways and underlying mechanisms. Knowledge-based Ingenuity Pathway Analysis alongside extensive literature research revealed that one key node of this network was PKA. As almost all proteins with the strongest changes in abundance (> 1.5-fold; LASP1, SKAP2, NAP1L1, CALD1, ZYX, and VASP) belonged to the cAMP/PKA-pathway, an involvement of post-translational modifications such as phosphorylation was anticipated. To provide biochemical evidence, protein lysates of PGI₂ and CTAD-treated platelets were treated with λ-phosphatase (λ-PPase).²⁸ Phosphorylated proteins lose their phospho-groups after λ-PPase treatment (treated+PPase/treated < 0.8), thereby causing a pI-shift towards an alkaline pH accompanied by an increase in the protein abundance of the corresponding "naïve" protein spots (treated+PPase/treated > 1.2). Using this approach, we were able to confirm phosphorylation of protein species due to pI-dependent abundance changes (Table 1A). Following the addition of λ-PPase (Figure 6), phosphorylation patterns were detected for VASP (pl 7.19-9.05), LASP1 (pl 6.34-6.83), alongside other potential PKA substrates as SKAP2 and ZYX (Figure 7). On the other hand, activation-dependent proteins appeared to belong mostly to the phospholipase C/protein kinase C (PKC-) pathway. Platelet activation by ADP and TRAP-6 induced significant abundance changes in MYL9, ILK and PLEK. The λ-PPase treatment also confirmed the phosphorylation of these protein spots (Table 1B). In addition, some phosphorylation sites could be validated by mass spectrometry.

To reveal phosphorylation of protein spots additionally to the λ-PPase treatment, the phosphorylation of Zyxin (ZYX) protein spots were validated by mass spectrometry (MS). Specifically, two identical peptides for the two ZYX-spots at pI 6.33 and pI 6.50, with additional phosphorylated sites on Ser169, Thr469, Ser505, and Ser545 at pI 6.33 were detected. Protein extracts of platelets stimulated with prostacyclin (PGI2) were separated in two-dimensions and gels were subsequently labeled by a mass spectrometry compatible silver stain. The ZYX protein spots with an isoelectric point (pi) of 6.33 and 6.50 were isolated and digested with trypsin. Thereby generated peptides were analysed by HPLC-coupled mass spectrometry and identified with the MASCOT protein database (http://www.matrixscience.com/search_form_select.html. accessed 16.01.2018). ZYX protein species pI 6.33 and pI 6.50 shared several peptides, howerer, they differed in their phosphorylation status.

To reveal phosphorylation of protein spots additionally to the λ-PPase treatment, the phosphorylation of Pleckstrin (PLEK) protein spots were validated by mass spectrometry (MS). Upon activation, one identical peptide for the two PLEK spots at pI 6.52 and pI 6.60 with two additional phosphorylation sides on Thr54 and Ser53 at pI 6.52 was detected. Protein extracts of platelets stimulated with ADP (adenosine-diphosphate) were separated in two-dimensions and gels were subsequently labeled by a mass spectrometry compatible silver stain. The PLEK protein spots with an isoelectric point (pI) of 6.60 and 6.52 were isolated and digested with trypsin. Thereby generated peptides were analysed by HPLC-coupled mass spectrometry and identified with the MASCOT protein database (http://www.matrixscience.com/search_form_select.html, accessed 16.01.2018). PLEK protein species pI 6.60 and pI 6.52 had an identical peptide, however, it differed in its phosphorylation status.

In conclusion, it appears that phosphorylation is a predominant feature in defining platelet inactivation. Based on these findings, it would be useful to identify further phosphorylation targets analogous to the established inactivation marker VASP.

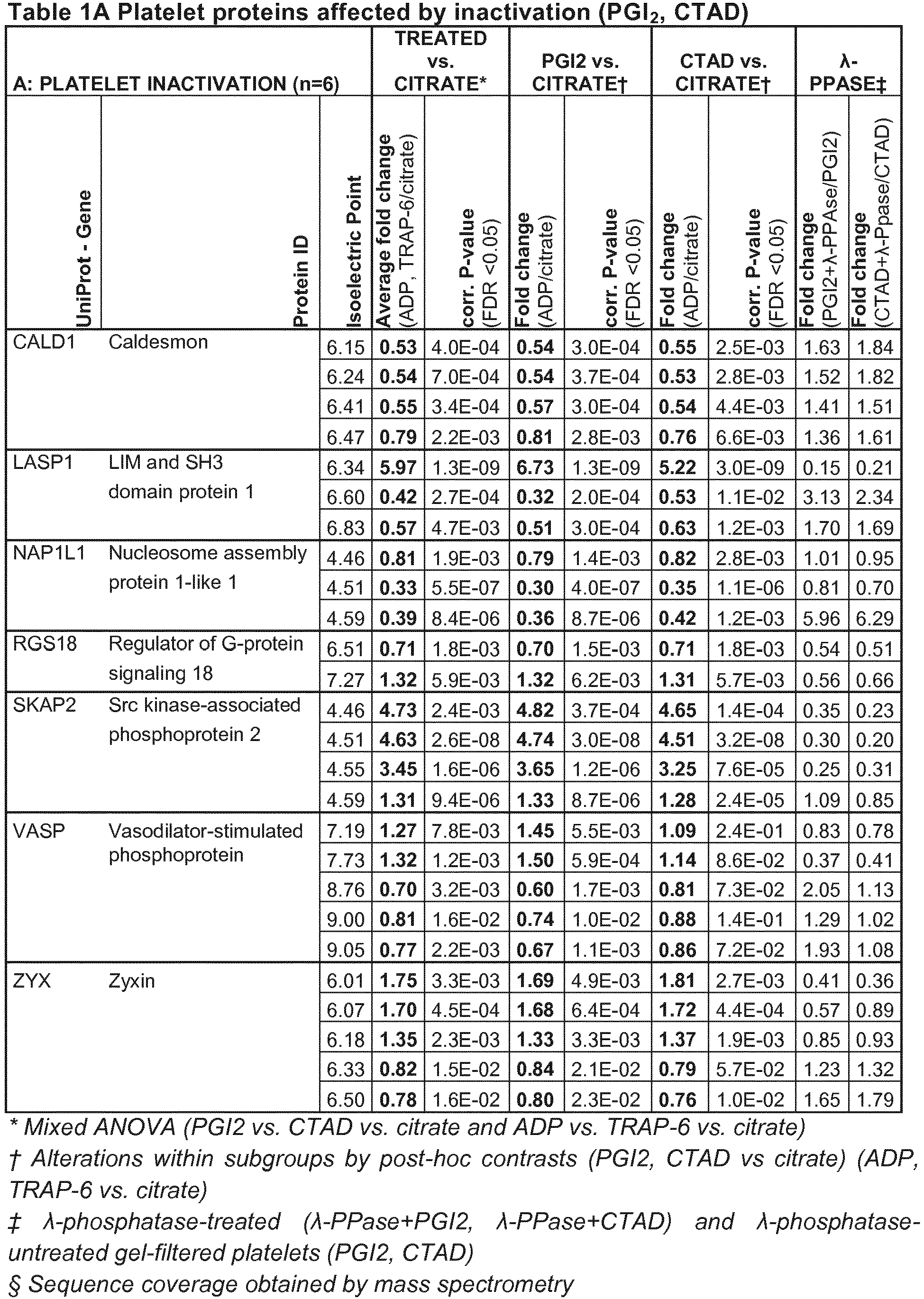

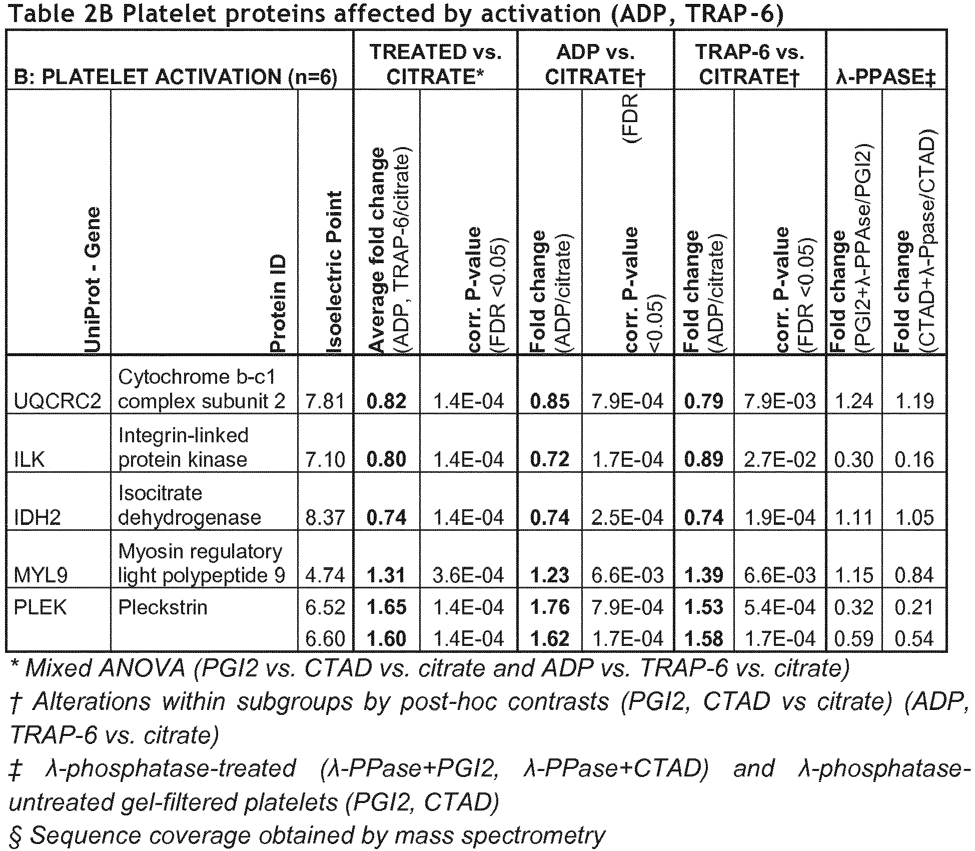

The tables (Table 1A and 1B) summarize alphabetically (Protein-ID) altered 2D-DIGE protein spots with their corresponding protein abundance changes upon (A) inactivation (PGI₂/CTAD) and (B) activation (ADP/TRAP-6). Alterations are given as average fold changes between subgroups (PGI₂, CTAD, ADP, TRAP-6) and their respective citrated untreated control (baseline). P-values corrected for multiple comparisons were obtained by post-hoc testing between the respective treatment subgroup and citrated controls. The sample size of each subgroup was n = 6. Proteins of interest were defined as (a) matched in 90% of all gels and (b) average fold change > 20% between at least one treatment condition and citrate (PGI₂ and/or CTAD vs. citrate; ADP and/or TRAP-6 vs. citrate) and c) a FDR-corrected p-value <0.05 obtained by a mixed ANOVA (PGI₂ vs. CTAD vs. citrate and ADP vs. TRAP-6 vs. citrate). Fold changes between λ-phosphatase-treated (λ-PPase+PGI₂, λ-PPase+CTAD) and - untreated (PGI₂, CTAD) samples are given to outline the protein phosphorylation status (n = 1). Fold changes > 20% between λ-phosphatase-treated and -untreated samples are considered as relevantly phosphorylated.

### 2D-DIGE analysis of inactivated platelets reveals more sensitive PKA targets than VASP

To evaluate the phosphorylation degree, protein abundances of phosphorylation-sensitive protein spots were compared between PGI₂/CTAD and citrate. Notably, 15 out of 26 inactivation-affected proteins originated from two or more protein spots, only differing in their pI. λ-PPase treatment confirmed that phosphorylation is responsible for this phenomenon. VASP, the most frequently described phosphorylation target of PKA/PKG, was affected in its phosphorylation profile by PGI₂ treatment (+1.45-FC at pI 7.19; p = 5.5E-03 and +1.50-FC at pI 7.73; p = 5.9E-04). CTAD treatment, on the other hand, did not significantly change the abundance of phosphorylated VASP protein species (+1.09-FC at pI 7.19; p = 2.4E-01 and +1.14-FC at pI 7.73; p = 8.6E-02) (Table 1A, Figure 5&8). These findings were also validated by flow cytometry (Figure 2). Most of the other potential PKA-targets, however, showed stronger abundance changes in their phosphorylated protein species than VASP, both by PGI2- and CTAD-treatment, like LASP1 (pI 6.34, +6.73-FC after PGI₂ and +5.22-FC after CTAD), SKAP2 (pI 4.46, +4.82-FC and +4.65-FC) and ZYX (pI 6.07, +1.68-FC and +1.72-FC) (Table 1A, Figure 5&8). For LASP1 (pI 6.34) and SKAP2 (pI 4.46), these changes were still present 25 minutes after gel-filtration (Figure 9). Thus, we demonstrated that phosphorylated LASP1, ZYX and SKAP2 species showed stronger protein abundance changes than phosphorylated VASP. Additionally, we were also interested in identifying protein markers for activation and in comparing them to the established activation marker CD62P.

### The Platelet Activation Marker CD62P is Strongly Associated with Phosphorylated Species of PLEK and ILK

After ADP or TRAP-6-mediated platelet activation, a considerably lower number of seven significantly changed protein spots were detected in the non-secretory proteome compared to inactivation (Figure 4). For example, PLEK, the major substrate of PKC, was detected in two protein spots (pI 6.52 and pI 6.60) and λ-PPase treatment confirmed their phosphorylation (Table 1B). Interestingly, ILK, in addition to PLEK, were the only two proteins which were affected in their phosphorylation status by both activation and inactivation. PLEK at pI 6.60 was phosphorylated upon activation, which was less pronounced upon inactivation when compared to the citrated control (Table 1B, Figure 10A). Inversely, ILK at pl 7.10 was dephosphorylated upon activation and phosphorylated after inactivation (Figure 10B). To assess whether this inverse manifestation in both systems is related to the activation marker CD62P, we evaluated their association (Figure 10 C&D). Despite a potent effect size of 2.35 (Cohen's d) for CD62P, the quantitative discrimination power between activation and inactivation was even more pronounced for PLEK (3.77 Cohen's d, pl 6.60) and ILK (3.79 Cohen's d, pl 7.10). These results show that ILK and PLEK are superior markers for platelet activation when compared to the commonly used CD62P.

### c) Discussion

Heightened platelet reactivity may result in thrombosis and other vascular diseases. By investigating the platelet proteome after in vitro activation and inactivation, we attempted to uncover signs of disruptions in platelet reactivity. Here, we showed for the first time that platelet inactivation was associated with more pronounced alterations in the non-secretory proteome as compared to activation. Moreover, we provided novel evidence that these changes were largely due to phosphorylation, hinting at an important role of post-translational modifications in maintaining platelets in their desired state. By employing 2D-DIGE in combination with bioinformatics analysis, inactivated platelets were revealed to exhibit changes in PKA targets (e.g. LASP1) that were more sensitive to phosphorylation compared to the benchmark inactivation marker VASP. Additionally, phosphorylated species of PLEK and ILK were associated with CD62P surface expression and showed an even larger effect size than this established activation marker. Thus, we can conclude that the proteins that we identified in this study might represent superior biomarkers for platelet reactivity compared to the established ones.

In our representative 2D-DIGE platelet proteome analysis, comprising 5,906 protein spots, 66 and 7 protein spots were significantly changed in response to 15 minutes inactivation and activation stimuli, respectively. On a physiological basis, the disparity between inactivation and activation could be due to platelets being more responsive to inactivation stimuli. Potent platelet deactivation is constantly needed due to the exposure to shear stress, acting as a strong platelet activator.²⁹ Shear stress is permanently present under physiological conditions and is much more pronounced in pathophysiological conditions, e.g. carotid or coronary vessel stenosis.^{30,31} Additionally, the present findings also demonstrate that the addition of PGI₂ or CTAD, to obtain quiescence platelets, strongly influences the non-secretory platelet proteome. Thus, after inactivation, 15 out of 26 proteins were altered in their phosphorylation levels. Knowledge-based pathway analysis demonstrated that the unbiased 2D-DIGE proteomics technology accentuated highly specifically the cAMP-induced PKA-signaling pathway in PGI₂ or CTAD-treated platelets. Accordingly, we identified five protein spots of the well-known PKA phosphorylation target VASP - a negative regulator of platelet activation³² and a clinically established inactivation marker.³³⁻³⁵ Remarkably, CTAD-treated platelets did not show a significant phosphorylation in any detected VASP species. Intriguingly, other spots showed substantially stronger abundance changes in their phosphorylation levels than phosphorylated VASP species: LASP1 6.7-fold, SKAP2 4.6-fold, ZYX 1.7-fold and RGS18 1.5-fold compared to VASP with 1.3-fold.

Until now, SKAP2 is not shown as a target of PKA, but phosphorylation upon PGI₂ treatment in resting platelets has been reported.³⁶ These new findings show that e.g. phosphorylated LASP1, and SKAP2 are technically more robust biomarkers to measure the platelet inactivation status than phosphorylated VASP. A previous platelet phospho-proteome study employing gel-free MS analysis revealed 300 proteins with differential phosphorylation patterns between 10 to 60 seconds after stimulation with the stable PGI₂ analog iloprost.¹⁷ However, a moderate upregulation of VASP in comparison to stronger upregulation of LASP1, SKAP2, ZYX and RGS18 was not reported. They found one phosphorylated VASP peptide (Ser239) out of five that was 2.0-fold up-regulated after platelet inactivation, whilst the others remained unchanged.¹⁷ The PKA phosphorylation target LASP1 was not found to be more phosphorylated and RGS18 phosphorylation decreased. However, these discrepancies regarding the affected phosphorylation targets may be related to their earlier measuring points in addition to the different methodology employed.

In the present study, PLEK (pl 6.60) and ILK (pl 7.10) species were the only two proteins that were affected by activation and inactivation. Aside from this, a pronounced overlap in the proteome was not found. This seemed plausible since ADP and thrombin inhibit adenylyl cyclase activity via Gi and, therefore, the first step in the inhibitory cAMP/PKA pathway.³⁷ PKC-dependent phosphorylation of PLEK is a sensitive marker for platelet activation.³⁸ Although ILK is described as a substrate for PKC and PI3K³⁹ to induce platelet adhesion⁴⁰, we found an explicit dephosphorylation pattern for ILK (pl 7.10) in activated platelets which has not been reported yet. Despite a potent effect size of 2.35 (Cohen's d) for the established platelet activation marker CD62P, the quantitative discrimination power between activation and inactivation was even more pronounced for phosphorylated PLEK (3.77 Cohen's d; pl 6.60) and ILK (3.79 Cohen's d, pl 7.10) species. As platelet CD62P expression may decrease in severe clinical states of platelet activation⁴¹, potentially due to receptor- and microvesicle-shedding and/or platelet adhesion to endothelial cells and leukocytes, phosphorylated ILK and PLEK species represent attractive and more robust biomarkers for platelet activation.

### REFERENCES

1. Benjamin EJ, Blaha MJ, Chiuve SE, et al. Heart Disease and Stroke Statistics-2017 Update: A Report From the American Heart Association. Circulation. 2017;135(10):e146-e603.
2. Vu TK, Hung DT, Wheaton VI, Coughlin SR. Molecular cloning of a functional thrombin receptor reveals a novel proteolytic mechanism of receptor activation. Cell. 1991;64(6):1057-1068.
3. Fisher GJ, Bakshian S, Baldassare JJ. Activation of human platelets by ADP causes a rapid rise in cytosolic free calcium without hydrolysis of phosphatidylinositol-4,5-bisphosphate. Biochem Biophys Res Commun. 1985;129(3):958-964.
4. Smolenski A. Novel roles of cAMP/cGMP-dependent signaling in platelets. J Thromb Haemost. 2012;10(2):167-176.
5. Manrique RV, Manrique V. Platelet resistance to prostacyclin. Enhancement of the antiaggregatory effect of prostacyclin by pentoxifylline. Angiology. 1987;38(2 Pt 1):101-108.
6. Mehta J, Mehta P, Conti CR. Platelet function studies in coronary heart disease. IX. Increased platelet prostaglandin generation and abnormal platelet sensitivity to prostacyclin and endoperoxide analog in angina pectoris. Am J Cardiol. 1980;46(6):943-947.
7. Jin RC, Voetsch B, Loscalzo J. Endogenous mechanisms of inhibition of platelet function. Microcirculation. 2005;12(3):247-258.
8. Rajendran S, Chirkov YY. Platelet hyperaggregability: impaired responsiveness to nitric oxide ("platelet NO resistance") as a therapeutic target. Cardiovasc Drugs Ther. 2008;22(3):193-203.
9. Xu XR, Zhang D, Oswald BE, et al. Platelets are versatile cells: New discoveries in hemostasis, thrombosis, immune responses, tumor metastasis and beyond. Crit Rev Clin Lab Sci. 2016;53(6):409-430.
10. Michelson AD, Furman MI. Laboratory markers of platelet activation and their clinical significance. Curr Opin Hematol. 1999;6(5):342-348.
11. Tomer A. Platelet activation as a marker for in vivo prothrombotic activity: detection by flow cytometry. J Biol Regul Homeost Agents. 2004;18(2):172-177.
12. Kabbani SS, Watkins MW, Ashikaga T, et al. Platelet reactivity characterized prospectively: a determinant of outcome 90 days after percutaneous coronary intervention. Circulation. 2001;104(2):181-186.
13. Garcia A, Prabhakar S, Hughan S, et al. Differential proteome analysis of TRAP activated platelets: involvement of DOK-2 and phosphorylation of RGS proteins. Blood. 2004;103(6):2088-2095.
14. Schweigel H, Geiger J, Beck F, et al. Deciphering of ADP-induced, phosphotyrosine-dependent signaling networks in human platelets by Src-homology 2 region (SH2)-profiling. Proteomics. 2013;13(6):1016-1027.
15. Garcia A, Senis YA, Antrobus R, et al. A global proteomics approach identifies novel phosphorylated signaling proteins in GPVI-activated platelets: involvement of G6f, a novel platelet Grb2-binding membrane adapter. Proteomics. 2006;6(19):5332-5343.
16. Parguina AF, Alonso J, Rosa I, et al. A detailed proteomic analysis of rhodocytin activated platelets reveals novel clues on the CLEC-2 signalosome: implications for CLEC-2 signaling regulation. Blood. 2012;120(26):e117-126.
17. Beck F, Geiger J, Gambaryan S, et al. Time-resolved characterization of cAMP/PKA-dependent signaling reveals that platelet inhibition is a concerted process involving multiple signaling pathways. Blood. 2014;123(5):e1-e10.
18. Beck F, Geiger J, Gambaryan S, et al. Temporal quantitative phosphoproteomics of ADP stimulation reveals novel central nodes in platelet activation and inhibition. Blood. 2017;129(2):e1-e12.
19. Wijten P, van Holten T, Woo LL, et al. High precision platelet releasate definition by quantitative reversed protein profiling--brief report. Arterioscler Thromb Vasc Biol. 2013;33(7):1635-1638.
20. Neufeld M, Nowak-Gottl U, Junker R. Citrate-theophylline-adenine-dipyridamol buffer is preferable to citrate buffer as an anticoagulant for flow cytometric measurement of platelet activation. Clin Chem. 1999;45(11):2030-2033.
21. Bleijerveld OB, van Holten TC, Preisinger C, et al. Targeted phosphotyrosine profiling of glycoprotein VI signaling implicates oligophrenin-1 in platelet filopodia formation. Arterioscler Thromb Vasc Biol. 2013;33(7):1538-1543.
22. Majek P, Reicheltova Z, Stikarova J, Suttnar J, Sobotkova A, Dyr JE. Proteome changes in platelets activated by arachidonic acid, collagen, and thrombin. Proteome Sci. 2010;8:56.
23. Zellner M, Winkler W, Hayden H, et al. Quantitative validation of different protein precipitation methods in proteome analysis of blood platelets. Electrophoresis. 2005;26(12):2481-2489.
24. Schluter H, Apweiler R, Holzhutter HG, Jungblut PR. Finding one's way in proteomics: a protein species nomenclature. Chem Cent J. 2009;3:11.
25. Baumgartner R, Umlauf E, Veitinger M, et al. Identification and validation of platelet low biological variation proteins, superior to GAPDH, actin and tubulin, as tools in clinical proteomics. J Proteomics. 2013;94:540-551.
26. Rozanas CR, Loyland SM. Capabilities using 2-D DIGE in proteomics research: the new gold standard for 2-D gel electrophoresis. Methods Mol Biol. 2008;441:1-18.
27. Winkler W, Zellner M, Diestinger M, et al. Biological variation of the platelet proteome in the elderly population and its implication for biomarker research. Mol Cell Proteomics. 2008;7(1):193-203.
28. Raggiaschi R, Lorenzetto C, Diodato E, Caricasole A, Gotta S, Terstappen GC. Detection of phosphorylation patterns in rat cortical neurons by combining phosphatase treatment and DIGE technology. Proteomics. 2006;6(3):748-756.
29. O'Brien JR. Shear-induced platelet aggregation. Lancet. 1990;335(8691):711-713.
30. Uchiyama S, Yamazaki M, Maruyama S, et al. Shear-induced platelet aggregation in cerebral ischemia. Stroke. 1994;25(8):1547-1551.
31. Kawano K, Yoshino H, Aoki N, et al. Shear-induced platelet aggregation increases in patients with proximal and severe coronary artery stenosis. Clin Cardiol. 2002;25(4):154-160.
32. Walter U, Eigenthaler M, Geiger J, Reinhard M. Role of cyclic nucleotide-dependent protein kinases and their common substrate VASP in the regulation of human platelets. Adv Exp Med Biol. 1993;344:237-249.
33. Assinger A, Schmid W, Volf I. Decreased VASP phosphorylation in platelets of male and female smokers of young age. Platelets. 2010;21(8):596-603.
34. Schwarz UR, Geiger J, Walter U, Eigenthaler M. Flow cytometry analysis of intracellular VASP phosphorylation for the assessment of activating and inhibitory signal transduction pathways in human platelets-definition and detection of ticlopidine/clopidogrel effects. Thromb Haemost. 1999;82(3):1145-1152.
35. Siller-Matula JM, Panzer S, Jilma B. Reproducibility and standardized reporting of the vasodilator-stimulated phosphoprotein phosphorylation assay. Platelets. 2008;19(7):551-554.
36. Zimman A, Titz B, Komisopoulou E, Biswas S, Graeber TG, Podrez EA. Phosphoproteomic analysis of platelets activated by pro-thrombotic oxidized phospholipids and thrombin. PLoS One. 2014;9(1):e84488.
37. Yang J, Wu J, Jiang H, et al. Signaling through Gi family members in platelets. Redundancy and specificity in the regulation of adenylyl cyclase and other effectors. J Biol Chem. 2002;277(48):46035-46042.
38. Manganaro D, Consonni A, Guidetti GF, et al. Activation of phosphatidylinositol 3-kinase beta by the platelet collagen receptors integrin alpha2beta1 and GPVI: The role of Pyk2 and c-Cbl. Biochim Biophys Acta. 2015;1853(8):1879-1888.
39. Pasquet JM, Noury M, Nurden AT. Evidence that the platelet integrin alphallb beta3 is regulated by the integrin-linked kinase, ILK, in a PI3-kinase dependent pathway. Thromb Haemost. 2002;88(1):115-122.
40. Jones CI, Tucker KL, Sasikumar P, et al. Integrin-linked kinase regulates the rate of platelet activation and is essential for the formation of stable thrombi. J Thromb Haemost. 2014;12(8):1342-1352.
41. Riedl J, Kaider A, Marosi C, et al. Decreased platelet reactivity in patients with cancer is associated with high risk of venous thromboembolism and poor prognosis. Thromb Haemost. 2017;117(1):90-98.

## Claims

1. An *in vitro* method of identifying abnormal platelet reactivity potential of a subject, comprising the steps of:
i) providing a sample comprising the platelet proteome of said subject, and
ii) measuring the phosphorylation level of at least one member of the cAMP/ protein kinase A (PKA)-pathway and comparing the phosphorylation level of said at least one cAMP/PKA-pathway member with the phosphorylation level of the corresponding at least one cAMP/PKA-pathway member in a reference sample, and/or
iii) measuring the phosphorylation level of at least one member of the phospholipase C/protein kinase C (PKC)-pathway and comparing the phosphorylation level of said at least one phospholipase C/PKC-pathway member with the phosphorylation level of the corresponding at least one phospholipase C/PKC-pathway member in a reference sample,
wherein a difference in the phosphorylation level of at least 1.5 fold in ii) and/or iii) is indicative of abnormal platelet reactivity potential, specifically the abnormal platelet reactivity potential is correlated with vascular disease or bleeding disorder.

2. The *in vitro* method of claim 1, wherein the at least one member of the cAMP/PKA-pathway is selected from the group consisting of LIM and SH3 domain protein 1 (LASP1), Src kinase-associated phosphoprotein 2 (SKAP2), Zyxin (ZYX), regulator of G-protein signaling 18 (RGS18), Caldesmon 1 (CALD1) and Nucleosome assembly protein 1-like 1 (NAP1L1) and/or wherein the at least one member of the phospholipase C/PKC-pathway is selected from the group consisting of pleckstrin (PLEK) and integrin-linked protein kinase (ILK).

3. The *in vitro* method of claim 1 or 2, wherein a decrease of at least 1.5 fold in the phosphorylation level of any one or more of LASP1, SKAP2, ZYX and RGS18 and/or an increase of at least 1.5 fold in the phosphorylation level of CALD1 and/or NAP1L1 and/or an increase of at least 1.5 fold in the phosphorylation level of PLEK and/or a decrease of at least 1.5 fold in the phosphorylation level of ILK indicates increased platelet reactivity potential.

4. The *in vitro* method of claim 1 for the diagnosis of a vascular disorder of a subject, comprising the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample of a healthy subject or a pool of healthy subjects,
i) wherein a decrease of at least 1.5 fold in the phosphorylation level of any one or more of LASP1, SKAP2, ZYX and RGS18 indicates increased platelet reactivity potential,
ii) wherein an increase of at least 1.5 fold in the phosphorylation level of CALD1 and/or NAP1L1 indicates increased platelet reactivity potential, and
wherein increased platelet reactivity potential identifies the subject as being at risk of or having a vascular disorder.

5. The *in vitro* method of claim 1 for the identification of treatment success of a subject having a vascular disorder, comprising the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample, which is an earlier sample of said subject,
i) wherein an increase of at least 1.5 fold in the phosphorylation level of any one or more of LASP1, SKAP2, ZYX and RGS18 indicates decreased platelet reactivity potential,
iii) wherein a decrease of at least 1.5 fold in the phosphorylation level of CALD1 and/or NAP1L1 indicates decreased platelet reactivity potential, and
wherein decreased platelet reactivity potential is indicative of treatment success.

6. The *in vitro* method of claim 1 for the diagnosis of a vascular disorder of a subject, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample of a healthy subject or a pool of healthy subjects,
i) wherein an increase of at least 1.5 fold in the phosphorylation level of PLEK indicates increased platelet reactivity potential,
iii) wherein a decrease of at least 1.5 fold in the phosphorylation level of ILK indicates increased platelet reactivity potential, and
wherein increased platelet reactivity potential identifies the subject as being at risk of or having a vascular disorder.

7. The *in vitro* method of claim 1 for the identification of treatment success of a subject having a vascular disorder, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample, which is an earlier sample of said subject,
i) wherein a decrease of at least 1.5 fold in the phosphorylation level of PLEK indicates decreased platelet reactivity potential,
iii) wherein an increase of at least 1.5 fold in the phosphorylation level of ILK indicates decreased platelet reactivity potential, and
wherein decreased platelet reactivity potential is indicative of treatment success.

8. The *in vitro* method of claim 1 for the diagnosis of a bleeding disorder of a subject, comprising the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample of a healthy subject or a pool of healthy subjects,
i) wherein an increase of at least 1.5 fold in the phosphorylation level of any one or more of LASP1, SKAP2, ZYX and RGS18 indicates decreased platelet reactivity potential,
ii) wherein a decrease of at least 1.5 fold in the phosphorylation level of CALD1 and/or NAP1L1 indicates decreased platelet reactivity potential, and
wherein decreased platelet reactivity potential identifies the subject as being at risk of or having a bleeding disorder.

9. The *in vitro* method of claim 1 for the identification of treatment success of a subject having a bleeding disorder, comprising the steps of:
a) providing a sample comprising the platelet proteome of said subject,
b) measuring the phosphorylation level of at least one member of the cAMP/PKA-pathway selected from the group consisting of LASP1, SKAP2, ZYX, RGS18, CALD1 and NAP1L1,
c) comparing the phosphorylation level of said at least one cAMP/PKA-pathway member with the phosphorylation level of the corresponding at least one cAMP/PKA-pathway member in a reference sample, which is an earlier sample of said subject,
i) wherein a decrease of at least 1.5 fold in the phosphorylation level of any one or more of LASP1, SKAP2, ZYX and RGS18 indicates increased platelet reactivity potential,
ii) wherein an increase of at least 1.5 fold in the phosphorylation level of CALD1 and/or NAP1L1 indicates increased platelet reactivity potential, and
wherein increased platelet reactivity potential is indicative of treatment success.

10. The *in vitro* method of claim 1 for the diagnosis of a bleeding disorder of a subject, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample of a healthy subject or a pool of healthy subjects,
i) wherein a decrease of at least 1.5 fold in the phosphorylation level of PLEK indicates decreased platelet reactivity potential,
iii) wherein an increase of at least 1.5 fold in the phosphorylation level of ILK indicates decreased platelet reactivity potential, and
wherein decreased platelet reactivity potential identifies the subject as being at risk of or having a bleeding disorder.

11. The *in vitro* method of claim 1 for the identification of treatment success of a subject having a bleeding disorder, comprising the steps of:
a) providing a sample comprising the platelet proteome of said subject,
b) measuring the phosphorylation level of at least one member of the phospholipase C/PKC-pathway selected from the group consisting of PLEK and ILK,
c) comparing the phosphorylation level of said at least one phospholipase C/PKC-pathway member with the phosphorylation level of the corresponding at least one phospholipase C/PKC-pathway member in a reference sample, which is an earlier sample of said subject,
i) wherein an increase of at least 1.5 fold in the phosphorylation level of PLEK indicates increased platelet reactivity potential,
ii) wherein a decrease of at least 1.5 fold in the phosphorylation level of ILK indicates increased platelet reactivity potential, and
wherein increased platelet reactivity potential is indicative of treatment success.

12. The *in vitro* method of any of claims 9 to 11, wherein the bleeding disorder is selected from the group consisting of hemophilia A, hemophilia B, factor I deficiency, factor II deficiency, factor V deficiency, factor VII deficiency, factor X deficiency, factor XI deficiency, factor XII deficiency, factor XIII deficiency and von Willebrand's disease.

13. The *in vitro* method of any of claims 4 to 8, wherein the vascular disease is selected from the group consisting of coronary artery disease, coronary atherosclerosis, myocardial infarction, stroke, thrombosis, coronary occlusion, hypertensive heart disease, hypertension, peripheral artery disease and diabetes, specifically type II diabetes.

14. The *in vitro* method of any of claims 1 to 13, wherein the sample is selected from the group consisting of whole blood, plasma, platelet rich plasma (PRP) and platelet enriched fraction, and/or wherein the reference sample is an earlier sample of the same subject or a sample of a healthy subject or of a pool of healthy subjects.

15. A kit of parts comprising
a) capturing reagents, preferably antibodies, capable of capturing at least one of phosphorylated and/or unphosphorylated LASP1, SKAP2, ZYX, RGS18, CALD, NAP1L1, PLEK and ILK, and
b) optionally, a reference sample, and
c) detection reagents for the detection of the phosphorylated and/or unphosphorylated proteins of a), and
d) an instruction leaflet comprising information on the correlation of increased or decreased phosphorylation levels of at least one of LASP1, SKAP2, ZYX, RGS18, CALD1, NAP1L1, PLEK and ILK with increased or decreased platelet reactivity potential corresponding to vascular disease and bleeding disorder.
